(19) Europäisches Patentamt
European Patent Office
Office européen des brevets

(11) EP 2 246 064 B1

(12) **EUROPEAN PATENT SPECIFICATION**

(45) Date of publication and mention
of the grant of the patent:
**08.08.2018 Bulletin 2018/32**

(51) Int Cl.:
*A61K 38/16* (2006.01)    *A61P 35/00* (2006.01)
*A61P 37/02* (2006.01)    *A61P 37/06* (2006.01)
*C07K 14/375* (2006.01)

(21) Application number: **08871843.2**

(22) Date of filing: **30.12.2008**

(86) International application number:
**PCT/CN2008/002142**

(87) International publication number:
**WO 2009/094850 (06.08.2009 Gazette 2009/32)**

(54) **RECOMBINANT GANODERMA LUCIDIUM IMMUNOMODULATORY PROTEIN (RLZ-8) AND USES THEREOF**

REKOMBINANTES IMMUNMODULATIONSPROTEIN (RLZ-8) AUS GANODERMA LUCIDIUM UND VERWENDUNGEN DAVON

PROTÉINE IMMUNOMODULATRICE RECOMBINANTE DE REISHI (GANODERMA LUCIDIUM) (RLZ-8) ET SES UTILISATIONS

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **03.01.2008 CN 200810050206**

(43) Date of publication of application:
**03.11.2010 Bulletin 2010/44**

(73) Proprietors:
• **Sun, Fei**
**Fengxian**
**Shanghai**
**201418 (CN)**
• **Zhang, Xitian**
**Shanghai 201418 (CN)**

(72) Inventors:
• **Sun, Fei**
**Fengxian**
**Shanghai**
**201418 (CN)**
• **Zhang, Xitian**
**Shanghai 201418 (CN)**

(74) Representative: **Nony**
**11 rue Saint-Georges**
**75009 Paris (FR)**

(56) References cited:
EP-A1- 1 666 592        EP-A2- 1 800 690
CN-A- 1 939 532         CN-A- 101 205 553
JP-A- 2 124 899         US-A1- 2007 071 766

• **MULLER ET AL: "Ganoderma lucidum causes apoptosis in leukemia, lymphoma and multiple myeloma cells", LEUKEMIA RESEARCH, NEW YORK,NY, US, vol. 30, no. 7, 1 July 2006 (2006-07-01), pages 841-848, XP005482052, ISSN: 0145-2126, DOI: DOI:10.1016/J.LEUKRES.2005.12.004**
• **MURASUGI A ET AL: "Molecular Cloning of a cDNA and a Gene Encoding an Imunomodulatory Protein, Ling Zhi-8, from a Fungus, Ganoderma lucidum", JOURNAL OF BIOLOGICAL CHEMISTRY, AMERICAN SOCIETY FOR BIOCHEMISTRY AND MOLECULAR BIOLOGY, INC, US, vol. 266, no. 4, 5 February 1991 (1991-02-05), pages 2486-2493, XP002455839, ISSN: 0021-9258**
• **HEM VAN DER L G ET AL: "LING ZHI-8: STUDIES OF A NEW IMMUNOMODULATING AGENT", TRANSPLANTATION, WILLIAMS AND WILKINS, BALTIMORE US, vol. 60, no. 5, 15 September 1995 (1995-09-15), pages 438-443, XP009022200, ISSN: 0041-1337**

- LIN, Z. P. ET AL.: 'Structure and function study of fungal immunomodulatory protein (FIP).' JOURNAL OF LIAONING NORMAL UNIVERSITY vol. 29, no. 1, March 2006, ISSN 1000-1735 pages 84 - 87
- JIN, B. Q.: 'Cellular and Molecular Immunology.', 2001, SCIENCE PRESS, BEIJING, ISBN 7-03-009578-2 pages 165 - 167
- GUO, Q. ET AL.: 'The progress of fungal immunomodulatory protein pharmacology.' ANHUI MEDICAL AND PHARMACEUTICAL JOURNAL. vol. 11, no. 5, May 2007, ISSN 1009-6469 pages 385 - 387
- LIN, J. W. ET AL.: 'The progress of fungal immunomodulatory protein.' CHINESE JOURNAL OF IMMUNOLOGY. vol. 21, no. 6, 2005, ISSN 1000-484X pages 477 - 480
- KINO, K. ET AL.: 'Isolation and characterization of a new immunomodulatory protein, Ling Zhi-8 (LZ-8), from Ganoderma lucidium.' THE JOURNAL OF BIOLOGICAL CHEMISTRY. vol. 264, no. 1, 1989, pages 472 - 478, XP009091137
- LIN WEN-HUEI ET AL: "Dimerization of the N-terminal amphipathic alpha-helix domain of the fungal immunomodulatory protein from Ganoderma tsugae (Fip-gts) defined by a yeast two-hybrid system and site-directed mutagenesis", JOURNAL OF BIOLOGICAL CHEMISTRY, THE AMERICAN SOCIETY OF BIOLOGICAL CHEMISTS, INC, US, vol. 272, no. 32, 8 August 1997 (1997-08-08), pages 20044-20048, XP002532070, ISSN: 0021-9258, DOI: 10.1074/JBC.272.32.20044

**Description**

**FIELD OF TECHNOLOGY**

The present invention relates to medical uses of a Fungal immunomodulatory protein, in particular a recombinant Ganoderma Lucidum Immunoregulatory Protein having a particular spatial structure for indications such as tumor, hypoleukocytosis and allergic diseases, etc.

**BACKGROUND TECHNOLOGY**

In existent researching files, a specified kind of proteins come from *Ganoderma Lucidum* exert extensive immune regulatory activities including regulation, red cells agglutinate, gene modulation of adhesion molecule, suppressing allergy reaction and immune anti-tumor effect.

The separation and purification of small-molecule protein from the extractive of Ganoderma mycelium was done by a Japanese Kino and others in 1989 (Kohsuke Kino et al., J. Bio. Chem. 1989, 1:472-478), it was named LZ-8, its amino acid sequence and physiological activity of immunity was also tested which indicates that the sequence of protein of LZ-8 is made up of 110 amino acid residues, acetylation, the molecular weight is 12.4 KD, and the isoelectric point is 4.4.

The inventor has disclosed for the first time the crystal structure of recombinant Ganoderma Lucidum Immunoregulatory Protein, mainly comprising an important N-terminal domain needed for the formation of dimerization and a C-terminal FNIII domain. The said N-terminal domain is composed of an $\alpha$-helix (having an amino acid sequence of 2-SDTA-LIFRLAWDVK-15 and being consisted of 14 amino acids) and a $\beta$-strand (having an amino acid sequence of 16-KLSFD-20 and being consisted of 5 amino acids), wherein residues of Ser on $\alpha$-helix are blocked by acetylation. The N-terminal $\alpha$-helix and $\beta$-strand of one LZ-8 monomer form, together with the same domains of another LZ-8 monomer, an important dumb-bell-shaped dimmer-binding domain via domain swapping. The C-terminal FNIII domain belongs to the immunoglobulin-like sandwich structure and comprises $\beta$-sheets I and $\beta$-sheets II formed by $\beta$-strand A-B-E and $\beta$-strand G-F-C-D, respectively. Researching results indicated that the immunomodulatory function of the recombinant Ganoderma Lucidum Immunoregulatory Protein may be realized essentially by forming homodimers via N-terminal $\alpha$-helix A (researches showed that 13 N-terminal amino acid residues are quite essential for forming functional homodimers for Fip), bonding to lymphocytes via the said homodimers, and prompting the lymphocytes to secrete various cytokines via a series of information transfer.

Among the existing clinical drugs having effect of increase of the number of leukocyte, recombinant colony-stimulating factor (such as recombinant human granulocyte macrophage colony-stimulating factor) is the only gene recombinant protein preparation used for treating hypoleukocytosis or granulocytopenia caused by various reasons and hypoleukocytosis caused by bone marrow suppression treatment. Although years of clinical application have proved the notable effects of recombinant colony-stimulating factor, all product containing it are marked with adverse effects like "The common side effect you may experience is aching in the bones and muscles, fever, tetter, pruritus, abdominal pain, diarrhoea, etc. A small number of patients may experience dose-response after initial administration, including flushing, sweating, a drop in blood pressure, a drop in blood oxygen saturation. Serious but rare adverse effects include allergies, bronchospasm, heart failure, supraventricular tachycardia, capillary leak syndrome, cerebrovascular disease, insanity, convulsions, a drop in blood pressure, intracranial hypertension, pulmonary edema, etc".

The previous reports about LZ-8 suggested that it kill tumor cells through immune regulatory pathways. In this invention, inventor found that rLZ-8 could kill HL-60, NB4, K562 tumor cells directly rather than indirect immune regulatory pathways.

The major function of Ganoderma Lucidum Immune Regulatory Protein lies in that it stimulates the hyperplasia of peripheral lymphocytes and spleen cells, induces the macrophage both in human and animals to secrete various cell factors (as in interleukin, tumor necrosis factor and interferon, etc.), and defending and dispelling the infringement of the causative agent, safeguards and maintains the health and to achieve the immune regulatory function. This invention suggest that rlZ-8 could prevent systemic allergic reactions and immune rejection after organ transplantation effectively.

**THE CONTENT OF INVENTION**

It is an objective of the present invention to find out the crystal structure of recombinant Ganoderma Lucidum Immunoregulatory Protein (rLZ-8) and its new medical uses.

The invention falls into the field of biomedical engineering, involving medical uses of recombinant Ganoderma Lucidum Immunoregulatory Protein for anti-tumor, raising leucocyte and immune suppression.

The recombinant Ganoderma Lucidum Immunoregulatory Protein (rLZ-8) described herein is obtained by recombinant expression in *Pichia pastoris* via gene engineering technology, and the nucleotide sequence of the rLZ-8 is re-designed and artificially synthesized according to published gene sequence (M58032) of natural Ganoderma Lucidum Immunoregulatory Protein.

It is described herein for the first time the crystal structure of recombinant Ganoderma Lucidum Immunoregulatory Protein (rLZ-8) by X-ray diffraction. The conditions for culturing crystal of the said protein are: 1.75 M ammonium sulfate, 0.1 M Tris pH 6.0 and 6.4% PEG 400. A single crystal with a space group P32 was obtained by hanging drop vapor diffusion method. Crystal data was collected at 1.80Å resolution by X-ray diffractometer's diffraction data collection system. Structure analysis was performed using softwares such as CCP4, etc and a crystal structure was obtained via phase exchange. It was found there were four molecules in each asymmetric unit, wherein a dimmer was formed between two molecules by hydrophobic interaction between N-terminal α-helix and hydrogen bonds between antiparallel β-strand. C-terminal of a single molecule has a FNIII type immunoglobulin-like fold, the domain is composed of two β-sheets. It is inferred that there is a sugar binding site at surface and junction of the beat-sheets, respectively.

In *in vitro* anti-tumor test described herein, rLZ-8 of different concentrations, which was prepared using IMDM culture medium, was added to acute promyelocytic leukemia cell line NB4 and chronic myeloid leukemia cell line K562 cultured *in vitro,* it was obviously observed that rLZ-8 imposed killing effect on said two tumor cells, and experimental data of MTT method showed that drug effect was significantly dependent on dosage. In *in vivo* anti-tumor test of the present invention, purified rLZ-8 was continuously administrated to S180/H22-bearing mice for 10 days, ascites tumor-bearing mice were weighed daily and sacrificed by cervical dislocation, tumors were cut from mice that were subcutaneously inoculated with tumor and weighed, then tumor inhibition rate was calculated and the number of leukocytes in blood before and after administration of drug was measured. The results indicated that the inhibition of rLZ-8 on tumor was dependent on dosage. Both of *in vitro* and *in vivo* tests indicated that the rLZ-8 described herein has notable anti-tumor effects.

In the description, Fluorchrome (Fluorescein-5-Isothiocyanate, FITC) was used to label rLZ-8, formed FITC-rLZ-8, hatched it with Hl60 cells, rat myocardial tissues and rabbit chondrocytes, collected and washed cells, under fluorescence microscope observation, with FITC-rLZ-8 incubation of 1h and 6h the HL-60 cells, with strong green fluorescence, compared with rat myocardial tissues and rabbit chondrocytes, there is a significant difference. These results served to show that a certain receptor could be recognized by rLZ-8. It is inferred that the recognition mechanism may be related to Oligosaccharide Link on the surface of HL-60 cells, because there were two carbohydrate chain binding sites on C-terminal FNIII domain of rLZ-8, and any Oligosaccharide Links have never been found on normal cells' surface. Base on this, the results suggested that specific killing effect of rLZ-8 could be due to an association with recognizing the receptors on the surface of cells.

For further studying the anti-tumor mechanism, the apoptotic percentage of rLZ-8-treated K562 and Hl60 cells was measured by flow cytometry using propidium iodode (PI) stain and ANNEXIN V&FITC stain, the results drew the conclusion that apoptosis of cells is one of the ways for rLZ-8 to kill tumor cells.

The present specification discloses that rLZ-8 is effective in preventing and treating hypoleukocytosis. In treatment experiment on rats/mice suffering from hypoleukocytosis caused by cyclophosphamide, rLZ-8 prepared with normal saline was continuously administrated to hypoleukocytosis-suffering rat models caused by cyclophosphamide for 7 days, with Genlei™ Sci-max™ (Recombinant human granulocyte colony-stimulating factor injection) as positive drug, tail vein blood sampling was performed on the third and seventh day, the number of leukocytes in blood was measured, leukocytes number differences before and after treatment were compared, and the drug effect was analyzed. Compared with cyclophosphamide control group, the number of leukocytes in rats of rLZ-8 group was greatly increased on the third day of administration, the difference was very significant, and the number of leukocytes recovered to normal level on the seventh day of administration.

In treatment experiment on mice suffering from hypoleukocytosis caused by γ radiation, each group of mice, except for the normal control group, were intraperitoneally injected with isovolumetric normal saline, positive drug (Genlei™ Scimax™) and rLZ-8 in different doses from the same day of radiation. The radiation conditions were: 7.50 Gy, 180 mV, 15 mA, with a dose rate of 150 roentgen per minute. Tail vein blood sampling was performed on each group of mice before the administration and on the fifth, seventh and ninth days of radiation, and the number of leukocytes was counted. Seven days after radiation, the mice were weighed and sacrificed by cervical dislocation, spleens were collected and weighed, then spleen indexes were calculated. Thereafter, the spleens were fixed in Bouin solution, and the number hemopoieticfocus on surface of the spleens (CFU-S) was counted after 6 hours. In preventing experiment on mice suffering from hypoleukocytosis caused by γ radiation, drugs were administrated five days before radiation, and the following steps were the same with those in above described treatment experiment. Experimental data of both experiments indicated that rLZ-8 was effective in preventing and treating hypoleukocytosis caused by γ radiation.

The present description shows that rLZ-8 does not produce hemolytic effect, does not impose agglutination effect on erythrocyte of four human blood types and does not cause abnormal change to mice' bone marrow. The present invention relates to the use a recombinant Ganoderma Lucidum Immunoregulatory Protein (rLZ-8) as defined by the claims. In some embodiments, the said medicament comprises the recombinant Ganoderma Lucidum Immunoregulatory Protein (rLZ-8) and a pharmaceutically acceptable adjuvant. In some embodiments, the said medicament includes drug for external use injection. In some embodiments, injection includes freeze-dried powder injection and water injection.

The rLZ-8 of the present description can be used as an immunosuppressant. It was found in experiments that rLZ-8 was effective in inhibiting systematic anaphylactic reactions of mice, and did not cause positive reactions, such ashyperspasmia, reduction in activity or death, to mice irritated by BSA (Bovine Serum Albumin).

The present description discloses preparing a pharmaceutical preparation containing rLZ-8 as active ingredient, the pharmaceutical preparation is mainly parenterally-administrated preparation, such as normal injection, freeze dried injection, liposomes injection, target-delivery injection, etc, and may be prepared into gastrointestinally-administrated preparation, such as tablet, capsule, spray, gel, gel absorbent, oral medicine, suspension, instant medicine, patch, pill, pulvis, injection, infusion solution, suppository, diluted preparation, controlled release preparation, etc.

The content of rLZ-8 in the pharmaceutical preparation of the present description is 1 to 99%, with the remaining being pharmaceutically acceptable carrier selected from a group consisting of starch, dextrin, sucrose, lactin, celluloses, magnesium stearate, Tween 80, Carbomer, methylcellulose, sodium carboxymethylcellulose, sodium alginate, glycerin, gelatine, polyethylene glycol, thimerosal, propylparaben, methylparaben, ethylparaben, acetonechloroform, sodium benzoate, borax, bromo-geramine, sorbitol, propanediol isopropanol, laurocapram, isopropanol/propanediol (1:1), isopropanol/ (1:2) and isopropanol/propanediol (2:1), triethanolamine, sodium hydroxide, vaseline, stearic acid, liquid paraffin, lanolin, octadecanol, hexadecanol, glyceryl monostearate, sldium lauryl sulfate, S-60, peregal 0, mannitol, sorbitol, etc.

The pharmaceutical preparation of the present description can be prepared by conventional process in the field.

## DESCRIPTION TO MANUAL FIGURES

Figure 1. rLZ-8 crystal structure captions

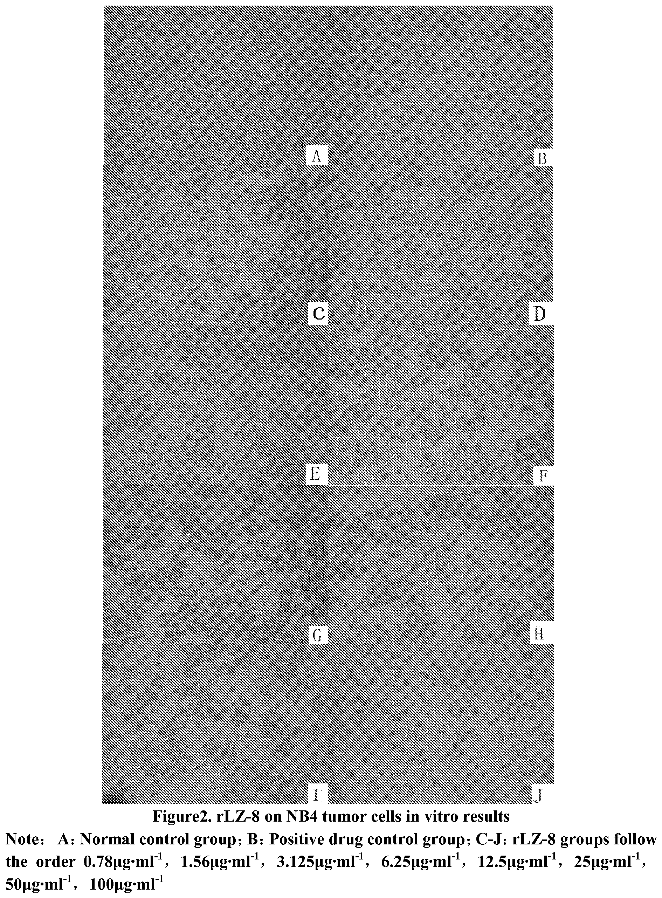

Figure2. rLZ-8 on NB4 tumor cells in vitro results

Figure 3. rLZ-8 on K562 tumor cells in vitro results

Figure 4. rLZ-8-induced apoptosis of K562 and NB4 cells, PI single staining test results

Figure 5 rLZ-8-induced apoptosis of H160 and NB4 cells, Annexin V/PI double staining test results

Figure 6 Inoculated S180 Ehrlich ascites tumor cells in mice body weight change

Figure 7 Inoculated H22 tumor cells implanted in mice body weight change

Figure 8. FITC-rLZ-8 (100ng.ml$^{-1}$) rat myocardial tissue markers (dark, DIC field)

Figure 9 FITC-rLZ-8 (100ng.ml$^{-1}$) rabbit chondrocyte markers (dark, DIC field)

Figure 10 FITC-rLZ-8 (100ng.ml$^{-1}$) HL-60 cell markers (dark, DIC field)

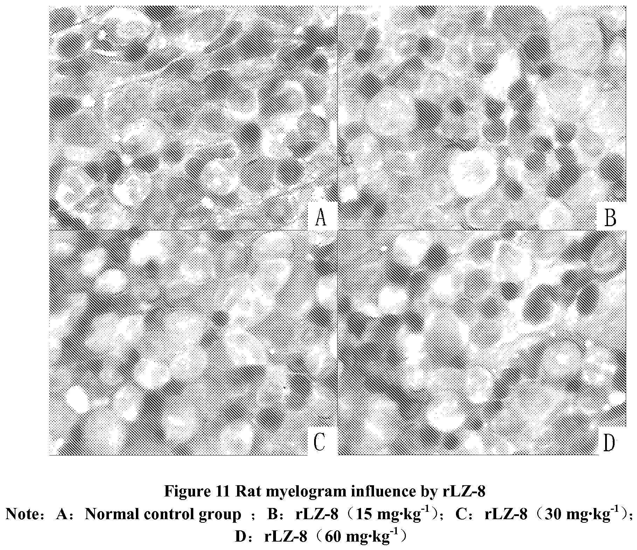

Figure 11 Rat myelogram influenced by rLZ-8

## EXAMPLES

### Example 1 Obtaining the Recombinant Ganoderma Lucidum Immune Regulatory Protein

### 1. Synthetic rLZ-8 genes, construction of engineering bacteria, construction and screening

In accordance with genetic code preference of *Pichia pastoris,* LZ-8 gene (SEQ1) was re-designed and synthesized for whole-gene based on the original Ganoderma Lucidum Immune immunomodulatory protein gene sequence (M58032), SEQ1 and M58032 ecode the same amino acid sequence, and the difference between them was that codon more suitable for expression system of *Pichia pastoris* was utilized in redesigned sequence.

The sequence (SEQ1) was linked with yeast α-factor leader peptide coding sequence to become a fusion gene, cloned α-LZ-8 gene into a pMD18-T carrier, linearized the carriers of correct sequencing and induced into yeast gene genome, and screening Mut$^+$ strains which efficiently utilizes methanol on the MM and MD plates.

The coding sequence of natural Ganoderma Lucidum Immunoregulatory Protein (M58032)

is: AGCATCATGTCCGACACTGCCTTGATCTTCAGGCTCGCCTGGGACGTGAAGAA-GCTCTCGTTCGACTACACCCGAACTGGGGCCGCGG-CAACCCCAACAACTTCATCGACACTGTCACCTTCCCGAAAGTCTTGAC-CGACAAGGCGTACACGTACCGCGTCGCCGTCTCCGGACGGAAC-CTCGGCGTGAAACCTCGTACGCGGTCGAGAGCGACGGCTCG-CAGAAGGTCAACTTCCTCGAGTACAACTCCGGGTATGGCATAGCGGACACGAACAC-GATCCAGGTGTTCGTTGTCGACCCCGACACCAACAACGACTTCATCATCGCCCAGTG-GAACTAGGAGGAGGCAG.

The nucleotide SEQUENCE (SEQ1) of redesigned recombinant Ganoderma Lucidum Immunoregulatory Protein (rLZ-8) is:

ATGTCTGATACTGCTTTGAT CTTCAGATTGGCTTGGGATGTTAAGAAGTTGTCTTTT-GATTACACTCCAAACTGGGGTAGAGGTAACCCAAACAACTTCATTGA-TACTGTTACTTTTCCTAAGGTTTTGACTGATAAGGCTTACACTTACAGAGTT-GCTGTTTCTGGTAGAAACTTGGGTGTTAAGCCATCTTACGCTGTT-GAATCTGATGGTTCTCAAAAGGTTAACTTCTTGGAATACAACTCTGGTTACGG-TATTGCTGATACTAACACTATTCAAGTTTTCGTTGTTGATCCAGATACTAACAACGAT-TTCATTATCGCTCAATGGAACTAGTAA.

The amino acid sequence of said recombinant Ganoderma Lucidum Immunoregulatory Protein (rLZ-8) is:

MSDTALIFRLAWDVKKLSFDYTPNWGRGNPNNFIDTVTFPKVLTDKAY-

TYRVAVSGRNLGVKPSYAVESDGSQKVNFLEYNSGYGIADTNTIQVF
VVDPDTNNDFIIAQWN;

### 2. The expression of rLZ-8 engineering bacteria

Expression of the fermentation scale, temperature, speed of rotation, pH values, liquid volume, supplement of methanol and other detections have established the optimized process of yeast in the 100L fermentor scale expression rLZ-8 conditions. We have designed the poilt fermentation medium formula in accordance with the physical and chemical properties of rLZ-8. The rLZ-8 output is about 800mg·L$^{-1}$.

### 3. Purification process of rLZ-8

Fermentation broth centrifugal separator → supernatant tubular separator → ultra filtration → cation exchange column purification → AKTA protein purification workstation for preparation of the target protein → strong anion-exchange chromatography purification → hydrophobic interaction column → gel filtration chromatography.

### 4. RLZ-8 Purity and molecular weight determination

Purity analysis of separation and purification using RP-HPLC, the purity of rLZ-8 is >99%. Evaluation of laser-flight mass spectrometry of Recombinant expression of rLZ-8 molecular weight is 12,722Da.

### 5. Determination of rLZ-8 higher spatial structure

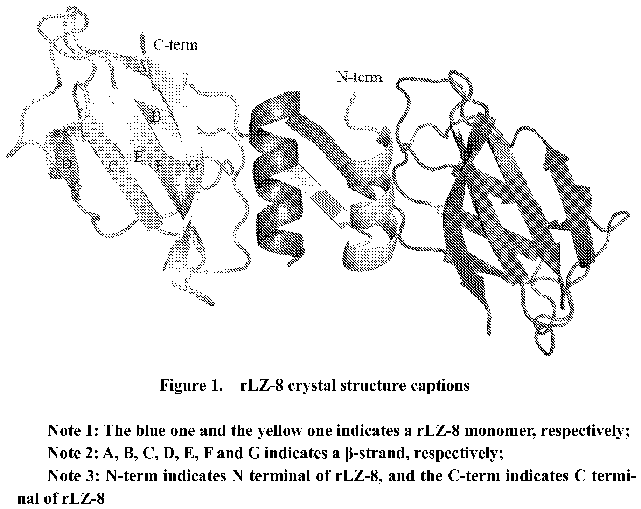

A single crystal with a space group P32 was obtained by hanging drop vapor diffusion method, with the culture condition being: 1.75 M ammonium sulfate, 0.1 M Tris pH 6.0 and 6.4% PEG 400. rLZ-8's X-ray diffraction data was collected at 1.80Å resolution by a MarResearch 345 dtd diffraction data collection system. Structure analysis was performed using softwares such as CCP4, etc and a crystal structure was obtained via phase exchange. The structure of the crystal rLZ-8 was briefly describes as following: the structure of LZ-8 comprising an important N-terminal domain needed for the formation of dimerization and a C-terminal FNIII domain. The N-terminal said domain is composed of an α-helix (having a amino acid sequence of 2-SDTA-LIFRLAW DVK-15 and being consisted of 14 amino acids) and a β-strand (having a sequence of 16-KLSFD-20 and being consisted of 5 amino acids), wherein residues of Ser on α-helix are blocked by acetylation. The N-terminal α-helix and β-strand of one LZ-8 monomer form, together with the same domains of another LZ-8 monomer, an important dumb-bell- shaped dimmer-binding domain via domain swapping. The C-terminal FNIII domain belongs to the immunoglobulin-like sandwich structure and comprises β-sheet I and β-sheet II formed, as shown in Fig.1, by β-strands A-B-E and β-strands G-F-C-D, respectively, wherein the sequence of β-strand was A. 21-TPNWGRG-27; B. 34-IDTVTFP-39; C. 48-YTYRVAV-54; D. 57-RNLGVKP-63; E. 72-SQKVN-76; F. 91-TIQVFVVDPD-100; G. 102-NNDFIIAQW-110.

**Example 2: Killing effect of rLZ-8 on human promyelocytic leukemia NB4 cells**

**1. Experimental Reagent**

After filtration and degerming, we prepared 8 concentrations using IMDM culture medium which are respectively 0.78μg·ml$^{-1}$, 1.56μg·ml$^{-1}$, 3.125μg·ml$^{-1}$, 6.25μg·ml$^{-1}$, 12.5μg·ml$^{-1}$, 25μg·ml$^{-1}$, 50μg·ml$^{-1}$, 100μg·ml$^{-1}$.

**2. Experimental Method**

On 96-orifice culture medium plate, the pilot orifice plus NB4 tumor cells 0.1ml and rLZ-8 0.1ml, rLZ-8 concentrates from low to high; negative control group plus NB4 tumor cells and culture medium of 0.1ml respectively; positive drug control group arsenic trioxide $As_2O_3$; make six-aperture multipunches for each group. Placing in 37°C, 5% $CO_2$ incubator 48h, adding MTT15μl (5mg ml$^{-1}$) prior to the termination of 4h, after the termination of cell culture by adding 100μl 0.1mol L$^{-1}$ hydrochloric acid isopropyl alcohol, testing on the enzyme-linked immuno-sorbent OD$_{570nm}$ values.

**3. Experimental Result**

Table 1 and Figure 1 showed that rLZ-8 drug group at OD$_{570nm}$ optical absorption value and NB4 normal control group, there are significant differences that rLZ-8 has a strong lethal effect in vitro on NB4 tumor cells.

**Table 1 rLZ-8's lethal effect in vitro on NB4 tumor cells ($\overline{X} \pm s$, n = 6)**

| Groups | Dosage (μg ml$^{-1}$) | OD$_{570nm}$ | Growth inhibition rate (%) |
|---|---|---|---|
| Normal control group Positive drug control group | - | 1.16±0.020 | - |
| rLZ-8 groups | 10 | 0.33±0.01* | 72 |
| | 0.78 | 0.71±0.03* | 39 |
| | 1.56 | 0.65±0.05* | 44 |
| | 3.125 | 0.53±0.04* | 54 |
| | 6.25 | 0.45±0.02* | 61 |
| | 12.5 | 0.30±0.04* | 74 |
| | 25 | 0.22±0.01* | 81 |
| | 50 | 0.11±0.01* | 91 |
| | 100 | 0.08±0.01* | 93 |

Comparison of drug group with NB4 normal control group, * *p*<0.01

**Example 3: Lethal effect of rLZ-8 on human chronic myelogenous leukemia K562 cells**

**1. Experimental Reagent**

After sterilization of rLZ-8, we prepared 6 concentrations using IMDM culture medium, which are respectively 3.125μg·ml$^{-1}$, 6.25μg·ml$^{-1}$, 12.5μg·ml$^{-1}$, 25μg ml$^{-1}$, 50μg·ml$^{-1}$, 100μg·ml$^{-1}$.

**2. Experimental Method**

On 96-orifice culture plate, the pilot aperture plus K562 tumor cells 0.1ml and rLZ-8 0.1ml, rLZ-8 concentrations from low to high; negative control group plus K562 tumor cells and culture medium of the 0.1ml respectively; positive drug control group arsenic trioxide $As_2O_3$; make six-aperture multipunches for each group. Placing in 37°C, 5% $CO_2$ incubator 48h, adding MTT15$\mu$l (5mg ml$^{-1}$) prior to the termination of 4h, after the termination of cell culture by adding 100$\mu$l 0.1mol L$^{-1}$ hydrochloric acid isopropyl alcohol, testing OD values $_{570nm}$ on the enzyme immunosorbent detector.

**3. Experimental Result**

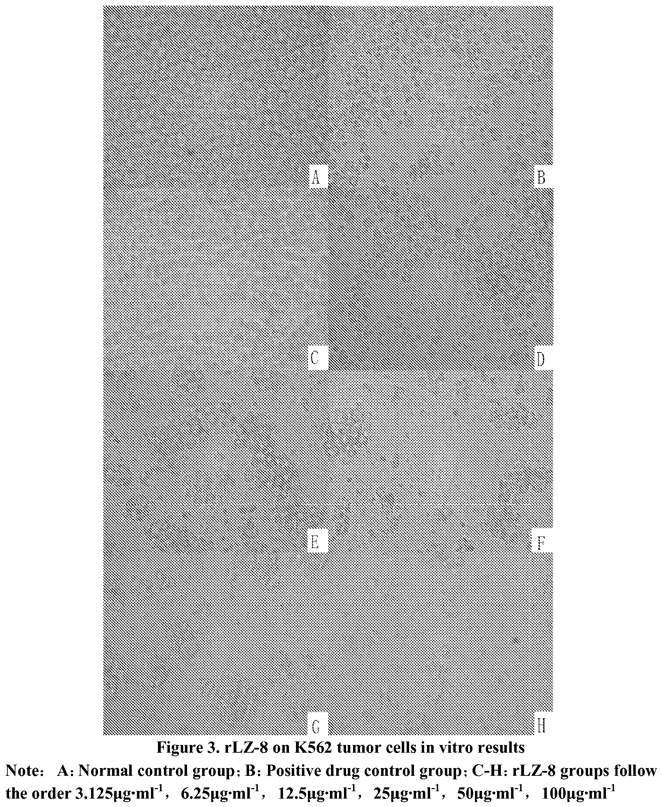

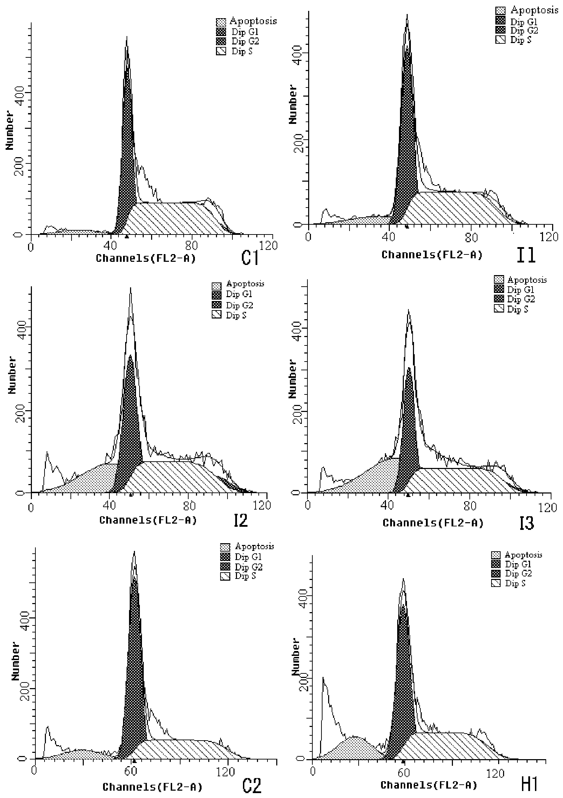

Table 2 and Figure 3 show that rLZ-8 drug group at $OD_{570nm}$ optical absorption value and K562 normal control group, there are significant differences that rLZ-8 has a strong lethal effect in vitro on K562 tumor cells.

**Table 2 rLZ-8 lethal effect on tumor cells K562 in vitro ($\overline{X}\pm$s, n=6)**

| Groups | Dosage ($\mu$g ml$^{-1}$) | $OD_{570nm}$ | Growth inhibition rate (%) |
|---|---|---|---|
| Normal control group Positive drug control group | - | 1.01±0.01 | - |
| rLZ-8 groups | 10 | 0.22±0.03* | 78.2 |
| | 3.125 | 0.66+0.03* | 34.7 |
| | 6.25 | 0.58-4-0.03* | 42.6 |
| | 12.5 | 0.52+0.05* | 48.5 |
| | 25 | 0.31±0.02* | 69.3 |
| | 50 | 0.25±0.04* | 75.2 |
| | 100 | 0.19±0.03* | 81.2 |

Comparison of drug groups with K562 normal control group, *$p$<0.01

**Example 4: rLZ-8 influence on apoptosis of blood tumor cell**

**1. PI single stained flow cytometry**

**1.1 Experimental Apparatus and cell line**

Fluorescence microscope, model Leica ASLMD, K562, NB4.

**1.2 Experimental Reagent**

rLZ-8 is classified into high, medium and low three-dose groups respectively, now we prepare them with IMDM culture fluid containing 2% FCS into the preparation of 2.5$\mu$g·ml$^{-1}$, 0.5$\mu$g/ml, 0.1$\mu$g·ml$^{-1}$. Propidium bromide (PI) 50$\mu$g·ml$^{-1}$.

**1.3 Experimental Grouping**

Establish K562 as the normal control group, positive drug control group ($As_2O_3$), rLZ-8 low-dose group (0.1$\mu$g·ml$^{-1}$), rLZ-8 medium dose group (0.5$\mu$g·ml$^{-1}$), rLZ-8 high-dose group, (2.5$\mu$g·ml$^{-1}$); set NB4 as the normal control group, rLZ-8 low-dose group (0.1$\mu$g·ml$^{-1}$), rLZ-8 medium dose group (0.5$\mu$g·ml$^{-1}$), rLZ-8 high-dose group, (2.5$\mu$g·ml$^{-1}$).

**1.4 Experimental Method**

Mix K562 and NB4 cells into 24-orifice plate respectively, and supply rLZ-8 in different concentrates, 1ml/orifice, set 3-orifice multipunches for each group. Put them in 37°C, 5% $CO_2$ incubator for 24h, collect the cells of each concentrate, PBS washes twice and regulates the cell density to $1\times10^6$/ml, 70% ice ethanol fixation, adjusts to -20°C and stays overnight. Wash the cells with PBS twice after fixing the cells, add PI (end density 50$\mu$g·ml$^{-1}$) for room temperature and lucifuged hatching 10min, 1000r.min$^{-1}$ centrifuge 5min, clear supernatant with disposable 400$\mu$ 1PBS re-suspension precipitation. And test on computer in 1h.

### 1.5 Experimental Result

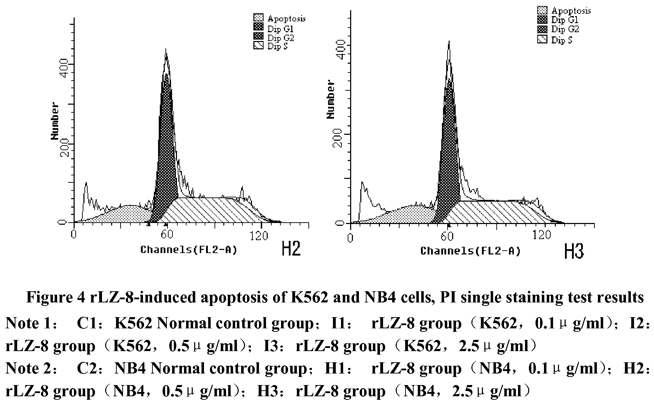

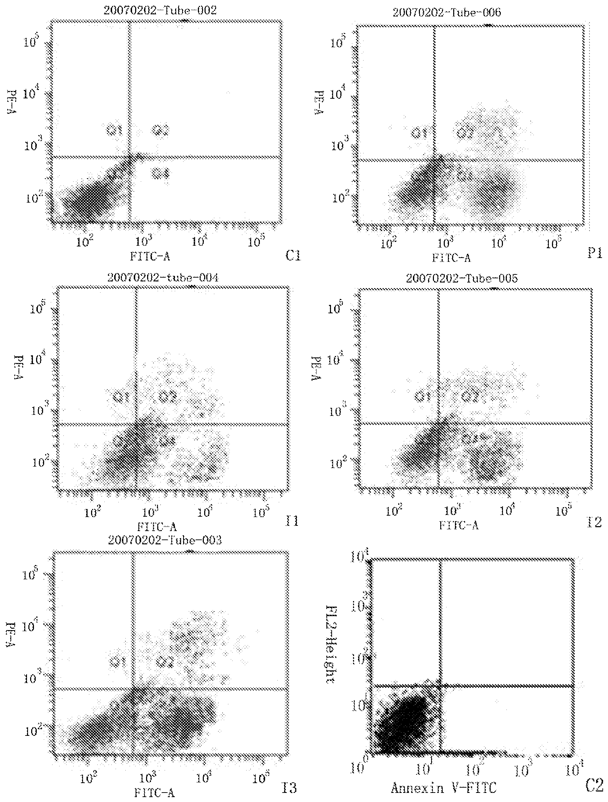

Table 3 and Figure 4 show that, comparison with K562 and NB4 normal control group, rLZ-8 drug group apoptosis rates increases, we may draw the conclusion that apoptosis of cells is one of the ways for rLZ-8 to kill tumor cells.

**Table 3 rLZ-8 induced apoptosis rate on K562 and NB4 (%)**

| Apoptosis rate | Normal contrast group | rLZ-8 (0.1μg·ml$^{-1}$) | rLZ-8 (0.5μg·ml$^1$) | rLZ-8 (2.5μg·ml$^{-1}$) |
|---|---|---|---|---|
| K562 | 8.89 | 16.43 | 17.91 | 21.57 |
| NB4 | 4.06 | 9.23 | 27.51 | 38.60 |

### 2. Annexin V-FITC Flow cytometry kit tests apoptosis of cells

### 2.1 Experimental Apparatus and cell line

FACS Calibur flow cytometry, U.S. Becton-Dickinson.Coompany. NB4, HL-60.

### 2.2 Experimental Reagent

Prepared rLZ-8 with IMDM culture fluid containing 2% FCS into the preparation of 0.1μg·ml$^{-1}$, 0.5μg·ml$^{-1}$, 2.5μg·ml$^{-1}$, arsenic trioxide (As$_2$O$_3$) 0.5μg·ml$^{-1}$ AnnexinV-FITC kit: combining buffer solution 4×; Propidium iodide solution (PI), 20μg·ml-1, 0.2ml.

### 2.3 Experimental Grouping

Establish NB4 normal control group, positive drug group (As$_2$O$_3$ 0.5μg·ml$^{-1}$), protein low-dose group (0.1μg·ml$^{-1}$), medium-dose group (0.5μg·ml$^{-1}$), high-dose group, (2.5μg·ml$^{-1}$); establish HL-60 normal control group, protein low-dose group (0.1μg·ml$^{-1}$), medium-dose group (0.5μg·ml$^{-1}$), high-dose group, (2.5μg·ml$^{-1}$).

### 2.4 Experimental Method

Mix NB4 and HL-60 cell into 24-orifice plate respectively, 1ml/hole supply rLZ-8 in different concentrates, set 3-orifice multipunches for each group. Put them in 37°C, 5% CO$_2$ incubator for 24h, collect the cells of each concentrate, washes the cells twice with pre-cooled PBS 4°C, with 250μl binding buffer solution re-suspending cells and regulates the cell density to 1×10$^6$/ml, taking out 100μl to 5ml streaming tube, and add 5μl Annexin V/FITC and 10μl 20μg·ml$^{-1}$ PI solution, after mixing put it in room temperature and lucifuged hatching 15min, add 400μl PBS by flow cytometry analysis.

### 2.5 Experimental Result

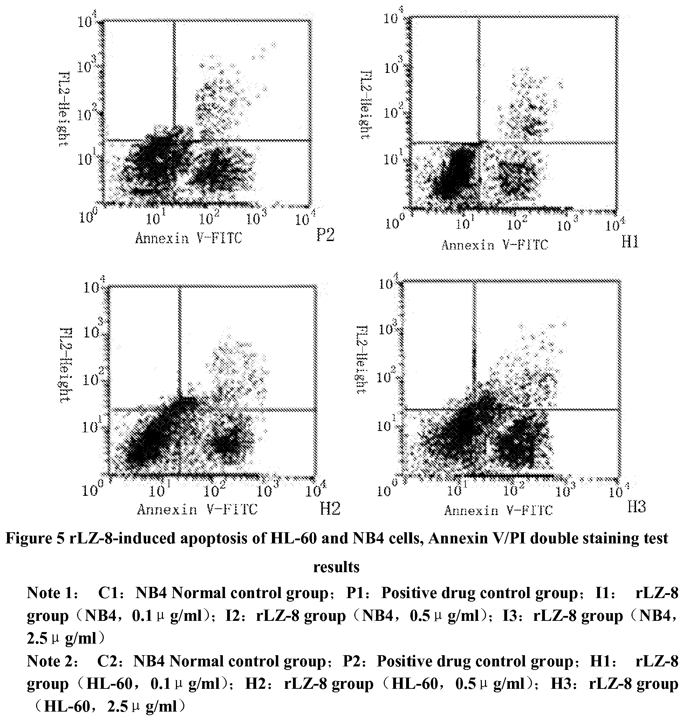

Figure 5 and Table 4 show that NB4-rLZ-8 group and HL-60-rLZ-8 group, the apoptosis rate is significantly higher than the normal control group, and, the apoptosis of HL-60-rLZ-8 group also rises with rLZ-8 concentration increase.

**Table 4 rLZ-8 inducible apoptosis rate on NB4 and HL-60 cell (%)**

| Apoptosis rate | Normal control group | Positive drug group | rLZ-8 (0.1μg-ml$^{-1}$) | rLZ-8 (0.5μg·ml$^{-1}$) | rLZ-8 (2.5μg·ml$^{-1}$) |
|---|---|---|---|---|---|
| NB4 | 6.1 | 23.4 | 21.0 | 22.3 | 34.0 |
| HL-60 | 0.4 | 39.7 | 30.5 | 40.7 | 47.7 |

### Example 5: rLZ-8 on mouse S180 Ehrlich ascites tumor inhibition

### 1. Experimental material

Mice, 18-22g, males and females were equally divided, provided by Jilin University Laboratory Animal Center; Mice Ehrlich ascites cells strain, provided by our laboratory S180; Cyclophosphamide (CTX), offered by Jiangsu Hengrui Medicine Co., Ltd., lot number: 06101921. S180 ascites tumors and solid tumors in experiment group were divided into normal control group, negative control group, positive control group, rLZ-8 low-dose treatment group (0.25mg·kg$^{-1}$), rLZ-8 medium-dose treatment group (0.5mg·kg$^{-1}$), rLZ-8 high-dose treatment group (1mg·kg$^{-1}$). For each group 10 mice.

### 2. Experimental Method

**S180 Subcutaneous Tumor Inhibition Experimental Method:** Select well-growing S180 cells, dilute appropriately with sterile saline into a tumor cell suspension, cell counts for $10^7 \cdot L^{-1}$, under the right armpit of each mouse inoculated subcutaneously 0.2ml (except for the normal control group). Treatment after 24h of vaccination. Normal control group and negative control group were given saline 0.2ml·each$^{-1}$·d$^{-1}$, intraperitoneal injection; positive control group were given cyclophosphamide 20mg·kg$^{-1}$, 0.2ml·each$^{-1}$·d$^{-1}$, intraperitoneal injection. rLZ-8 treatment groups were given doses of tail venous injection respectively, 0.2ml·each$^{-1}$·d$^{-1}$, in 10 consecutive days. Draw blood from orbital venous plexus 10 days before and after medication respectively, sent to Jilin University No.1 Hospital clinical laboratory to test and analyze the number of WBC. Kill all mice the next day of drug withdrawal at cervical dislocation, dissect anatomically and take out the tumors, weigh tumors, calculate the inhibition rate using the following equation:

$$\text{Inhibition rate (\%)} = (\text{average tumore weight of the control group} - \text{average tumore weight}$$
$$\text{of the experiment group})/\text{average tumor weight of the control group} \times 100\%$$

**S180 Ascites Tumor Inhibition Experimental Method:** Select well-growing S180 cells, dilute appropriately with sterile saline into a tumor cell suspension, cell counts for $10^7 \cdot L^{-1}$, each mouse intraperitoneal inoculation 0.2ml (except for the normal control group). Treatment after 24h of vaccination. Normal control group and negative control group were given saline 0.2ml·each$^{-1}$·d$^{-1}$, intraperitoneal injection; positive control group were given cyclophosphamide 20mg·kg$^{-1}$, 0.2ml·each$^{-1}$·d$^{-1}$, intraperitoneal injection. rLZ-8 treatment group were given doses of tail venous injection respectively, 0.2ml·each$^{-1}$·d$^{-1}$, in 10 consecutive days. Daily weighing, observe the weight changes of mice, drawing weight growth curves.

### 3. Experimental Result:

**S180 Subcutaneous Tumor Inhibition Experimental results:** As can be seen from Table 5, three doses of rLZ-8 can inhibit the growth of S180, inhibition rates are 16.8%, 25.7% and 45.5%. There are very significant differences ($p<0.01$), rLZ-8 treatment group tumor weight compared with the negative control group.

As can be seen from Table 6, before medication, the WBC numbers in mice are at the same level for all the groups, compared with the negative control group showed no differences ($p>0.05$). 10 days after the treatment, the negative control group WBC numbers were higher than the normal control group, rLZ-8 low-dose group and medium-dose group WBC numbers and negative control group showed no differences ($p>0.05$), high-dose group and the normal control group also without differences ($p>0.05$), the positive control group WBC numbers significantly decreased, and compared with normal control group and negative control group there were great differences ($p<0.01$).

**Table 5. rLZ-8 effect on mouse transplanted S180 tumor inhibition ($\overline{X}\pm$s, n=10)**

| Groups | Dose (mg·kg$^{-1}$) | Tumor weight (g) | Inhibition rate (%) |
|---|---|---|---|
| Negative control group | - | 1.01±0.03 | - |
| CTX group | 20 | 0.35±0.02* | 65.3 |
| rLZ-8 low-dose group | 0.25 | 0.84±0.03* | 16.8 |
| rLZ-8 medium-dose group | 0.5 | 0.75±0.02* | 25.7 |
| rLZ-8 high-dose group | 1 | 0.55±0.03* | 45.5 |
| Comparison with the negative control group, * $P < 0.01$ | | | |

**Table 6. rLZ-8 effect on mouse transplanted S180 interleukin ($\overline{X}\pm$s, n=10)**

| Groups | Dose (mg·kg$^{-1}$) | Number of leukocytes | |
|---|---|---|---|
| | | Before medication | 10th day of medication |
| Normal control group | - | 9.49±0.27 | 9.54±0.33 |
| Negative control group | - | 9.54±0.25 | 10.44±0.34 |

(continued)

| Groups | Dose (mg·kg⁻¹) | Number of leukocytes | |
| --- | --- | --- | --- |
| | | Before medication | 10th day of medication |
| CTX group | 20 | $9.56 \pm 0.31$* | $4.41 \pm 0.25$* |
| rLZ-8 low-dose group | 0.25 | $9.48 \pm 0.30$* | $10.44 \pm 0.18$** |
| rLZ-8 medium-dose group | 0.5 | $9.43 \pm 0.44$* | $10.34 \pm 0.31$** |
| rLZ-8 high-dose group | 1 | $9.49 \pm 0.36$* | $9.55 \pm 0.36$** |
| Comparison with the negative control group,* $p < 0.01$; ** $p > 0.05$ | | | |

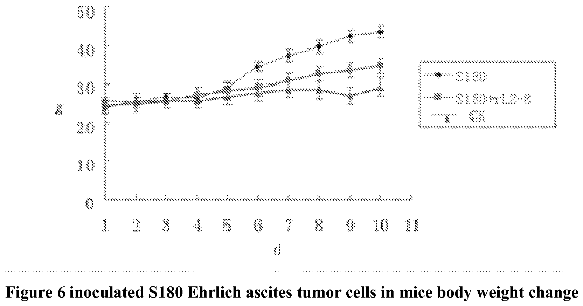

**S180 Ascites Tumor Inhibition Experimental results:** The experiment results showed that mice ascites in all the groups appeared basically at the same time, the negative control group of mice body weight increased rapidly, reduced survival time. From Fig.6 we could see that mice in rLZ-8 group the average weight of growth trend than the normal group, but more than the negative control group is relatively small. Note rLZ-8 to a certain extent, inhibited mouse peritoneal S180 tumor cells growth.

**Example 6: rLZ-8 on mouse hepatoma cell H22 inhibition experiment**

**1. Experimental material**

Mice, weight 18-22g, males and females were divided equally, from Jilin University Laboratory Animal Center. Mouse hepatoma cell strain H22, offered by our laboratory. Cyclophosphamide (CTX), offered by Jiangsu Hengrui Medicine Co., Ltd., lot number: 06101921.

**2. Experimental Method**

H22 hepatoma cells experiment group were divided into normal control group, negative control group, positive control group, rLZ-8 low-dose treatment group (0.25mg·kg⁻¹), rLZ-8 mei-dum-dose treatment group (0.5mg·kg⁻¹), rLZ-8 high-dose treatment group (1mg·kg⁻¹). For each group 10 mice.

**H22 Subcutaneous Tumor Inhibition Experimental Methods:** Select well-growing H22 cells, dilute appropriately with sterile saline into a tumor cell suspension, cell counts for $10^7 \cdot L^{-1}$, under the right armpit of each mouse inoculated subcutaneously 0.2ml (except for the normal control group). Treatment after 24h of vaccination. Normal control group and negative control group were given saline 0.2ml·each⁻¹·d⁻¹, intraperitoneal injection; positive control group were given cyclophosphamide 20mg·kg⁻¹, 0.2ml·each⁻¹·d⁻¹, intraperitoneal injection. rLZ-8 treatment groups were given doses of tail venous injection respectively, 0.2ml·each⁻¹·d⁻¹, in 10 consecutive days. Draw blood from orbital venous plexus 10 days before and after medication respectively, sent to Jilin University No.1 Hospital clinical laboratory to test and analyze the number of WBC. Kill all the mice the next day of drug withdrawal at cervical dislocation, dissected anatomically and took out the tumors, weigh tumors, calculated the inhibition rate using the following equation:

$$\text{Inhibition rate } (\%) = (\text{average tumore weight of the control group} - \text{average tumor weight}$$
$$\text{of the experiment group})/\text{average tumor weight of the control group} \times 100\%.$$

**H22 Ascites Tumor Inhibition Experimental Methods:** Select well-growing H22 cells, dilute appropriately with sterile saline into a tumor cell suspension, cell counts for $10^7 \cdot L^{-1}$, each mouse intraperitoneal inoculation 0.2ml (except for the normal control group). Treatment after 24h of vaccination. Normal control group and negative control group were given saline 0.2ml·each⁻¹·d⁻¹, intraperitoneal injection; positive control group were given cyclophosphamide 20mg·kg⁻¹, 0.2ml·each⁻¹·d⁻¹, intraperitoneal injection. rLZ-8 treatment groups were given doses of tail venous injection respectively, 0.2ml·each⁻¹·d⁻¹, in 10 consecutive days. Daily weighing, observe the weight changes of mice, drawing weight growth curves.

**3. Experimental Result:**

**H22 Subcutaneous Tumor Inhibition Experimental Result:** As can be seen from Table 7, all three rLZ-8 dose groups can inhibit the growth of S180, inhibition rates were 16.7%, 30.0%, 42.5%. There are very significant differences ($p < 0.01$), rLZ-8 treatment group tumor weight compared with the negative control group.

**Table 7 rLZ-8 effect on mouse transplanted inhibition of tumor H22 ($\overline{X}\pm$s, n=10)**

| Groups | Dosage (mg·kg$^{-1}$) | Tumor weight (g) | Inhibition rate (%) |
|---|---|---|---|
| Negative control group | - | 1.20±0.02 | - |
| CTX group | 20 | 0.45±0.02* | 62.5 |
| rLZ-8 low-dose group | 0.25 | 1.00±0.03* | 16.7 |
| rLZ-8 medium-dose group | 0.5 | 0.84±0.02* | 30.0 |
| rLZ-8 high-dose group | 1 | 0.69±0.03* | 42.5 |
| Comparison with the negative group, * $p < 0.01$ | | | |

From Table 8 we know that the WBC counts of mice in these groups before medication were at the same level, compared with the negative control group there showed no differences ($p> 0.05$). 10 days after treatment, the negative control group WBC counts were higher than that of the normal control group, rLZ-8 low-dose group and medium-dose group WBC ccounts and that of the negative control group showed no differences ($p> 0.05$), the high-dose group compared with the normal control group showed no differences ($p> 0.05$), the WBC counts in positive control group significantly decreased, compared with the normal control group and the negative control group were significantly different ($p<0.01$).

**Table 8 rLZ-8 effect on mouse transplanted inhibition of tumor H22 interleukin ($\overline{X}\pm$s, n=10**

| Groups | dosage (mg·kg$^{-1}$) | WBC numbers | |
|---|---|---|---|
| | | Before medication | 10$^{th}$ day of medication |
| Normal control group | - | 9.65±0.28 | 9.69±0.26 |
| Negative control group | - | 9.76±0.31 | 10.49±0.33 |
| CTX group | 20 | 9.67±0.43* | 4.49±0.21* |
| rLZ-8 low-dose group | 0.25 | 9.65±0.33* | 10.53±0.24** |
| rLZ-8 medium-dose group | 0.5 | 9.65±0.38* | 10.43±0.27** |
| rLZ-8 hig-dose group | 1 | 9.63±0.41* | 9.86±0.27** |
| Comparison with the negative group, * $p<0.01$; ** $p>0.05$ | | | |

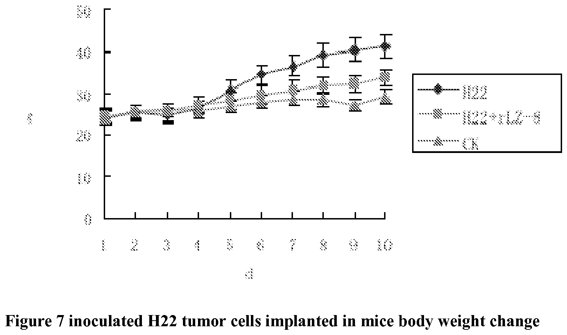

**H22 Ascites Tumor Inhibition Experimental Result:** The results showed rLZ-8 groups of mice survive longer than the negative control group, in the negative control group mice showed loss of appetite, but there is a rapid growth in weight and less activity. It can be seen from Fig.7 that rLZ-8 group, the growth weight of mice was bigger than the normal group on averge, but less than the negative control group. It indictes that rLZ-8 inhibited the mouse peritoneal H22 tumor cells growth of mouse to a certain extent.

**Example 7: rLZ-8 fluorescent labeling and its influence on normal tissue cells and HI-60 cells**

**1. rLz-8 fluorescence of FITC label**

**1.1 Experimental Reagent and Apparatus**

Fluorchrome (Fluorescein- 5-Isothiocyanate, FITC), GL Biochem (Shanghai); Dimethyl sulfoxide; carbonate buffer solution (pH 8~9.5) (Na$_2$CO$_3$ 4.3g, NaHCO$_3$ 8.6g add ddH$_2$O to 500ml); Phosphorus buffered saline (PBS); Desalting Hiprep 26/10 desalting column; AKTA purifier; spectrophotometer as Hitachi model.

**1.2 Experimental Method**

Mix the purified rLZ-8 (7.5mg·ml$^{-1}$) 20ml with carbonate buffer (pH8.3), for dialysis overnight, weighing 3.75mg FITC, add dimethyl sulfoxide (DMSO) 3.75ml into FITC-DMSO solution. First put rLZ-8 in a small 50ml beaker and then by mixing FITC-DMSO solution drops into rLZ-8 solution, and increase with PBS to 30ml, stirring 4h with magnetic stirrer at room temperature and lucifuging, desalting column with Desalting Hiprep 26/10 in AKTA purifier system to remove free fluorescein, 75ml PBS elution, 280nm, 495nm detection, and peak collection.

**1.3 Experimental Result:**

Scan the prepared FITC-rLZ-8 (10-times diluted) at 220nm~520nm, A495 = 0.445, A280 = 0.67, calculate tag efficiency (F/P) 3.80.

## 2. The labeling effect on rat myocardial tissues

### 2.1 Experimental Reagent and Apparatus

Leica CM1850 frozen slicer; wistar rat; fluorescence microscope 80i (Nikon); isotonic PBS buffer (pH7.2); fetal bovine serum (FBS, Gibco); FITC-rLZ-8 is prepared by our laboratory.

### 2.2 Experimental Method

Cut the rats at neck and kill them, and strip the hearts, put them to frozen microtome till the temperature dropped to -20°C for slicing, hatching for 1h at 37°C when the myocaridial and PBS preparation of FITC-rLZ-8 solution (100ng·ml$^{-1}$), observing under fluorescence microscope to observe, and establish a blank control group.

### 2.3 Experimental Result:

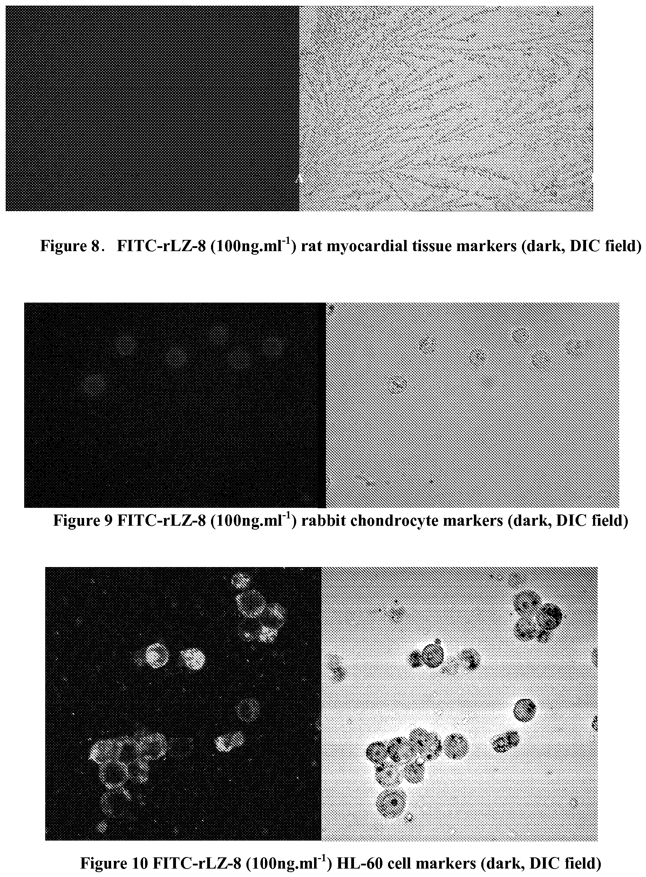

Under fluorescence microscope observation of myocardial tissues, it is without visible fluorescence, compared with the blank control group, there was no difference, see figure 8.

## 3. Labeling effect on primary culture of rabbit chondrocytes

### 3.1 Experimental Reagent and Apparatus

Japanese white rabbits (male, 2.5kg) 4; surgical instruments; 0.25% trypsogen +0.02% EDTA; 0.2% collagenase ‖ ; D-Hanks; IMDM medium (50ml·ml$^{-1}$, vitamin C, double-antibody); 0.025mg·ml$^{-1}$ Poly-lysine solution; sterile water for injection (WFI).

### 3.2 Experimental Method

Fix the experiment animals and execute them with air embolism, skinned abdominal in the middle and exposed the limbs, cutting the muscle fascia with scissors, disconnected the backbone from os longum, remove carefully the entire knee, hip and shoulder bones, with a rough pruning, immersed into D-Hanks. Moved the beaker with tissues into the super-clean units, move the tissues after pruning and cleansing into the 2nd sterile D-Hanks cup; assemble the knife, cutting a thin layer of cartilage off, use curved forceps move into the 6cm culture incubator, washing with D-Hanks three times, discard majority of D-Hanks, cut the cartilage into 1mm slices with ophthalmic scissors, discard majority of D-Hanks, spoon removing the broken bone pieces into a10cm$^2$ culture flask, mix with trypsogen EDTA for digestion, 37°C, 30min; replace the trypsogen for collagenase, put it into the hatching incubator of 37°C, taking out and shaking 5min every 1h, with 4-4.5h, finish digestion. Prepare it to the cell suspension solution and mix 3ml IMDM containing FBS 15%, $5 \times 10^4$·ml$^{-1}$ vaccinated into the culture flask.

Vaccinated the cell on the 24-orifice plate, 0.5ml/hole, establish a blank control group, hatching at 37°C for 1h with the final concentration of 0.25$\mu$g·ml$^{-1}$ FITC-rLZ-8 0.5ml., move the cells into 1.5ml EP centrifugal (1000r·min$^{-1}$, 7min) washing with isotonic PBS thre times, mix EP tube with 0.1ml PBS, re-suspended the cells, check the suspension to make observation under the fluorescenece mircroscope.

### 3.3 Experimental Result:

Observation under fluorescence microscope, the chondrocytes form of rabbits intact, without green fluorescence, compared with the control group there was no significant difference. Photos of experiment group are shown as in Figure 9.

**4. Labeling effect of HI-60 cells**

**4.1 Experimental Reagent and Apparatus**

Fluorescence microscope 80i (Nikon), IMDM cell culture medium (Hyclone), fetal bovine serum (FBS, Gibco), FITC-rLZ-8 and isotonic PBS buffer (pH7.2) Prepared by our laboratory.

**4.2 Experimental Method**

Vaccinated the HL-60 by $2 \times 10^6$ inoculation on 24-hole plate, each hole 0.5ml, with IMDM (2% FBS) culture medium prepared FITC-rLZ-8 for 100ng·ml$^{-1}$, each hole 0.5ml hatching (37°C), establish a control group, experiment group 1h and experiment group 6h, draw the cells respectively from 1h and 6h, mixing into 1.5mlEP tube, 1000r·min$^{-1}$ centrifuge, clear the supernatant, wash with PBS 3 times, re-suspend after washing.

**4.3 Experimental Result**

Figure 10 observe under fluorescence microscope, with FITC-rLZ-8 incubation of 1h and 6h the HL-60 cells, with strong green fluorescence, and in group 6h there was agglutination of cells, while in the blank control group, no green fluorescence, compared with former, there is a significant difference.

**Example 8: rLZ-8 Effect in the prevention of the leucocyte in mice**

**1. Medicine Preparation**

rLZ-8 preparation of sterile saline. Divide them into 5 μg·kg$^{-1}$, 2.5 μg·kg$^{-1}$, 1.25 μg·kg$^{-1}$, 0.62 μg·kg$^{-1}$ dose groups, 0.2ml/piece.
Genlei™ Scimax™ [Recombinant human granulocyte colony-stimulating factor injection (rhG-CSF)], production lot number: 20060403; 75μg/piece was prepared with sterile normal saline into the preparation of 3.2μg·kg$^{-1}$, 0.2ml/piece.
Cyclophosphamide (CTX) for Injection (the production lot number 07121821; 200mg / vial) was prepared with sterile normal saline into the preparation of 12.5 mg·kg$^{-1}$, 0.2ml/piece.
3% solution of acetic acid is prepared by taking 3 ml glacial acetic acid and adding $H_2O$ to 100ml, then filtering the resultant solution.

**2 Experimental Method**

The mice were divided into 7 groups, and each group comprises 5 male mice and 5 female mice. Except for the mice of the normal control group administrated with identical volume of sterile normal saline, each mouse of each group was continuously administrated 0.2 ml solution of cyclophosphamide (12.5 mg·kg$^{-1}$) through intraperitoneal injection per day for 3 days. Tail vein blood sampling was performed on the third day, the number of leukocytes in blood was measured under microscope . After successful modeling, except for the mice of the normal control group and the mice of group treated with cyclophosphamide (CTX) administrated with identical volume of sterile normal saline, said each mouse of each group was treated with conresponing dose of rLZ-8 and positive drugs (Genlei™ Sc imax™). Venous blood of tail is collected on the third, senventh, fourteenth day of the treatment respectively. Leukocytes number differences before and after treatment were compared, and the drug effect was analyzed.
The method for counting the number of leukocyte: the blood is diluted 20-fold with 3% solution of acetic acid so as to make erythrocyte dissolve. The resultant solution was dropped into leukocyte count cell, and counted the number of leukocyte under microscope, and caculated the number of leukocyte/mm$^3$.

**3 Experimental Result**

As can be seen from Table 9, compared with the CTX group, treatment on the 3rd day of rLZ-8 drug group in mice had significantly higher WBC, the difference is very significant, it recovers on the 7th day of treatment.

**Table 9 rLZ-8 Effect on low-interleukin mice model ($\overline{X} \pm$s, n=10)**

| grouping | Before modeling | After modeling | 3rd day of medication | 7th day of medication | 14th day of medication |
|---|---|---|---|---|---|
| Normal control group | 10.5±0.20 | 11.0±0.25 | 10.8±0.51 | 10.2±0.11 | 11.2±0.31 |

(continued)

| grouping | Before modeling | After modeling | 3rd day of medication | 7th day of medication | 14th day of medication |
|---|---|---|---|---|---|
| CTX group | 10.7±0.56 | 1.5±0.71 | 4.6±0.34 | 8.3±0.34 | 12.0±0.32 |
| Genlei™ Scimax™ (3.2µg·kg-1) | 10.8±0.22 | 1.5±0.35 | 5.5±0.12* | 22.7±0.12* | 13.0±0.19* |
| rLZ-8 (5µg·kg-1) | 9.92±0.21 | 1.3±0.32 | 5.0±0.12* | 20.3±0.11* | 14.3±0.21* |
| rLZ-8 (2.5µg·kg-1) | 10.25±0.39 | 1.5±0.54 | 6.6±0.77* | 22.8±0.15* | 15.2±0.11* |
| rLZ-8 (1.25µg·kg-1) | 10.4±0.31 | 1.7±0.45 | 8.1±0.17* | 19.7±0.17* | 13.9±0.14* |
| rLZ-8 (0.62µg·kg-1) | 10.4±0.91 | 1.4±0.45 | 6.4±0.41* | 27.2±0.10* | 13.9±0.17* |

Comparison between CP control group, * P <0.01

## Example 9

### rLZ-8 Effect in the treatment of the leucocyte in rats

### 1. Medicine Preparation

rLZ-8 preparation of sterile saline. Divide them into 20µg·kg-1, 10 µg·kg-1, 5 µg·kg-1, 2.5 µg·kg-1, 1.25 µg·kg-1, 0.625 µg·kg-1, 0.31 µg·kg-1 dose groups.

Genlei™ Scimax™ [Recombinant human granulocyte colony-stimulating factor injection (rhG-CSF)], production lot: 20071104; 150µg/piece was prepared with sterile normal saline into the preparation of 9.45µg·ml-1, 0.1ml/piece.

Cyclophosphamide (CTX) for injection (the production lot number 07121821; 200mg/vial) was prepared with sterile normal saline to 49 mg·kg-1, 0.2ml/vial

3% solution of acetic acid is prepared by taking 3 ml glacial acetic acid and adding $dH_2O$ to 100ml, then filtering the resultant solution.

### 2 Experimental Method

The rats were divided into 10 groups, and each group comprises 5 male rats and 5 female rats. Except for the rats of the normal control group administrated with identical volume of sterile normal saline, each rat of each group was continuously administrated 0.2 ml solution of cyclophosphamide (49 mg·kg-1) through intraperitoneal injection per day for 3 days. Tail vein blood sampling was performed on the third day, the number of leukocytes in blood was counted under microscope. After successful modeling, except for the rats of the normal control group and the rats of group treated with cyclophosphamide (CTX) administrated with identical volume of sterile normal saline, said each rat of each group was treated with conresponing dose of rLZ-8 and positive drugs (Genlei™ Sc imax™). Venous blood of tail is collected on the third, senventh, fourteenth day of the treatment respectively. Leukocytes number differences before and after treatment were compared, and the drug effect was analyzed.

The method for counting the number of leukocyte: the blood is diluted 20-fold with 3% solution of acetic acid so as to make erythrocyte dissolve. The resultant solution was dropped into leukocyte count cell, and counted the number of leukocyte under microscope, and caculated the number of leukocyte/$mm^3$.

### 3 Experimental Result

As can be seen from Table 10, compared with the CTX group, treatment on the 3rd day of rLZ-8 drug group in rats had significantly higher WBC, the difference is very significant, it has returned to normal on the 7th day of treatment.

**Table10 rLZ-8 Effect on low-interleukin rat model ($\overline{X}$±s, n=10)**

| grouping | Before modeling | After modeling | 3rd day of medication | 7th day of medication | 14th day of medication |
|---|---|---|---|---|---|
| Normal control group | 11.8±0.21 | 11.2±0.41 | 10.5±0.29 | 11.0±0.85 | 12.0±0.11 |
| CTX group | 11.56±0.89 | 0.8±0.71 | 0.6±0.32 | 11.2±0.12 | 11.5±0.12 |
| Genlei™ Scimax™ (9.45µg·kg-1) | 11.7±0.14 | 0.6±0.23 | 1.4±0.11* | 13.1±0.21* | 10.3±0.14 |

(continued)

| grouping | Before modeling | After modeling | 3rd day of medication | 7th day of medication | 14th day of medication |
|---|---|---|---|---|---|
| rLZ-8 (20 $\mu$g·kg$^{-1}$) | 11.2±0.11 | 0.7±0.34 | 1.6±0.33* | 15.8±0.13* | 9.8±0.19* |
| rLZ-8 (10 $\mu$g·kg$^{-1}$) | 11.1±0.34 | 0.6±0.56 | 1.6±0.71* | 15.8±0.33* | 10.9±0.21* |
| rLZ-8 (5 $\mu$g·kg$^{-1}$) | 11.4±0.22 | 0.8±0.79 | 2.6±0.64* | 17.6±0.23* | 11.2±0.26 |
| rLZ-8 (2.5 $\mu$g·kg$^{-1}$) | 11.0±0.98 | 0.8±0.12 | 1.7±0.18* | 12.8±0.11* | 10.6±0.29* |
| rLZ-8 (1.25 $\mu$g·kg$^{-1}$) | 12.0±0.24 | 0.7±0.11 | 2.8±0.12* | 11.0±0.47* | 10.3±0.45* |
| rLZ-8 (0.62 $\mu$g·kg$^{-1}$) | 11.7±0.45 | 0.8±0.74 | 5.2±0.14* | 12.8±0.74* | 12.1±0.24* |
| rLZ-8(0.31 $\mu$g·kg$^{-1}$) | 14.2±0.11 | 0.9±0.12 | 1.9±0.17* | 13.5±0.13* | 11.0±0.31 |
| Comparison between CP control group, * $p$<0.01 | | | | | |

## Example 10

### rLZ-8 Effect in the prevention of model of mice by radiation

### 1. Medicine Preparation

rLZ-8 preparation of sterile saline. Divide them into 5$\mu$g·kg$^{-1}$, 2.5$\mu$g·kg$^{-1}$, 1.25 $\mu$g·kg$^{-1}$, 0.62$\mu$g·kg$^{-1}$ dose groups. 0.2ml/piece.

Genlei™ Scimax™ [Recombinant human granulocyte colony-stimulating factor injection (rhG-CSF)], the production lot: 20060403; 150$\mu$g/piece was prepared with sterile normal saline into the preparation of 3.2 $\mu$g·kg$^{-1}$, 0.2ml/vial.

cyclophosphamide (CTX) for injection (the production lot number 07121821; 200mg/vial) was prepared with sterile normal saline to 12.5 mg·kg$^{-1}$, 0.2ml/vial.

### 2 Experimental Method

The mice were divided into 7 groups, and each group comprises 5 male mice and 5 female mice. Except for the mice of the normal control group administrated with identical volume of sterile normal saline, each mouse of each group was continuously administrated positive drugs (Genlei™ Sc imax™) and rLZ-8 in different doses once a day for 5 days. Radiation was performed on the fifth day, and the radiation conditions were: 7.50 Gy, 180 mV, 15 mA, with a dose rate of 150 roentgen per minute. Tail vein blood sampling was performed on each group of mice before the administration and on the fifth day of administration, and on the fifth day and the seventh day of radiation, and the number of leukocytes was counted. The method for counting the number of leukocyte is the same as above described. The mice were weighed and sacrificed by cervical dislocation, spleens were collected and weighed, then spleen indexs were calculated. Thereafter, the spleens were fixed in Bouin solution, and the number hemopoieticfocus on surface of the spleens (CFU-S) was counted after 6 hours.

### 3 Experimental Result

As can be seen from table 11, the number of leukocytes of the mice of the groups treated with rLZ-8 in different dosehas increased significantly on the fifth day of administration compared with CTX model group, and a significant difference exists between CTX group and rLZ-8 groups (p <0.05). The numbers of leukocytes in rLZ-8 groups were the lowest on the fifth day of radiation and increased on the seventh day, and a significant difference exists between mode group and rLZ-8 groups (p <0.05).

**Table 11. The results of effect in the prevention of model of mice by radiation ($\overline{X}$±s, n=10)**

| Group | Before medicine | 5th day | 5th day after radiation | 7th day after radiation |
|---|---|---|---|---|
| Normal control | 11.5±0.20 | 11.0±0.25 | 10.8±0.21 | 9.8±0.11 |
| Model control | 11.5±0.56 | 14.4±0.71 | 0.30±0.33 | 0.26±0.33 |
| Genlei™ Scimax™ 3.2$\mu$g·kg$^{-1}$ | 11.4±0.22 | 21.8±0.35* | 0.26±0.20 | 0.22±0.12 |
| rLZ-8 (5 $\mu$g·kg$^{-1}$) | 10.3±0.21 | 19.5±0.32* | 0.32±0.18 | 0.53±0.35* |
| rLZ-8 (2.5 $\mu$g·kg$^{-1}$) | 11.7±0.39 | 12.8±0.54* | 0.32±0.15 | 0.45±0.20* |
| rLZ-8 (1.25 $\mu$g·kg$^{-1}$) | 10.4±0.31 | 12.5±0.45* | 0.25±0.11 | 0.46±0.11* |

(continued)

| Group | Before medicine | 5th day | 5th day after radiation | 7th day after radiation |
|---|---|---|---|---|
| rLZ-8 (0.62 μg·kg⁻¹) | 11.2±0.91 | 10.7±0.45* | 0.16±0.31 | 0.40±0.12* |

Comparison between model group, * *p*<0.05

## Example 11

### rLZ-8 Effect in the treatment of model of mice by radiation

### 1. Medicine Preparation

rLZ-8 preparation of sterile saline. Divide them into 5μg·kg⁻¹, 2.5μg·kg⁻¹, 1.25 μg·kg⁻¹, 0.62μg·kg⁻¹ dose groups. 0.2ml/piece.

Genlei™ Scimax™ [Recombinant human granulocyte colony-stimulating factor injection (rhG-CSF)], production lot: 20060403; 150μg/vial, was prepared with Sterile normal saline to 3.2 μg·kg⁻¹, 0.2ml/ vial.

Injection of cyclophosphamide (CP) (the production lot number 07121821; 200mg/vial) was prepared with sterile normal saline into the preparation of 12.5 mg·kg-1, 0.2ml/vial.

### 2 Experimental Method

The mice were divided into 7 groups, and each group comprises 5 male mice and 5 female mice. Each group of mice, except for the normal control group, were intraperitoneally injected with isovolumetric normal saline, positive drug (Genlei™ Scimax™) and rLZ-8 in different doses from the same day of radiation. The radiation conditions were: 7.50 Gy, 180 mV, 15 mA, with a dose rate of 150 roentgen per minute. Tail vein blood sampling was performed on each group of mice before the administration and on the fifth, seventh and ninth days of radiation, and the number of leukocytes was counted. Seven days after radiation, the mice were weighed and sacrificed by cervical dislocation, spleens were collected and weighed, and then spleen factors were calculated. Thereafter, the spleens were fixed in Bouin solution, and the number hemopoieticfocus on surface of the spleens (CFU-S) was counted after 6 hours.

### 3 Experimental Results

As can be seen from table 12, the numbers of leukocytes in rLZ-8 groups were the lowest on the seventh day of radiation and increased on the ninth day, and a significant difference exists between mode group and rLZ-8 groups (p <0.05).

As can be seen from table 13, all rLZ-8 groups have stimulated hyperplasia of spleen growth. The spleen factor and colony forming unit-spleen (CFU-S) of mice of each rLZ-8 group were higher than those of mice of model group. (p<0.05)

**Table 12. The results of effect in the treatment of model of mice by radiation ($\overline{X}$±s, n=10**

| Group | Before medicine | 5th day | 7th day | 9th day |
|---|---|---|---|---|
| Normal control | 10.5±0.20 | 11.0±0.25 | 10.0±0.25 | 12.8±0.51 |
| Model control | 11.0±0.56 | 0.7±0.71 | 0.2±0.71 | 0.61±0.01 |
| Genlei™ Scimax™ (3-2μg·kg⁻¹) | 11.9±0.22 | 0.68±0.35 | 0.58±0.35* | 0.80±0.11* |
| rLz-8 (5μg·kg⁻¹) | 11.2±0.21 | 0.84±0.32* | 0.31±0.32* | 0.90±0.14* |
| rLz-8 (2.50μg·kg⁻¹) | 12.9±0.39 | 0.69±0.54 | 0.56±0.54* | 0.54±0.13* |
| rLz-8 (1.25μg·kg⁻¹) | 9.6±0.31 | 0.82±0.45* | 0.65±0.45* | 0.70±0.12* |
| rLz-8 (0.62μg·kg⁻¹) | 10.4±0.91 | 0.73±0.43* | 0.26±0.43* | 0.64±0.01 |

Comparison between model group, * *p*<0.05

**Table 13. The number of colony forming unit-spleen in model of mice by radiation ($\overline{X}\pm$s, n=10)**

| Group | Number | Factors | Growth rate |
|---|---|---|---|
| Normal contrast Genlei™ Scimax™ | 4.4±0.10 | 0.17±0.11 | |
| (3.2μg·kg$^{-1}$) | 6.0±0.11* | 0.20±0.10* | 26% |
| rLz-8 (5μg·kg$^{-1}$) | 9.7±0.16* | 0.22±0.09 | 54% |
| rLz-8 (2.50μg·kg$^{-1}$) | 14.3±0.03* | 0.52±0.12* | 69% |
| rLz-8 (1.25μg·kg$^{-1}$) | 6.3±0.21* | 0.24±0.13 | 30% |
| rLz-8 (0.62μg·kg$^{-1}$) | 8.9±0.41* | 0.50±0.10* | 50% |
| Comparison between model group, * $p<0.01$ | | | |

**Example 12: rLZ-8 inhibiting systemic allergic reaction in mice**

**1. Experimental Reagent**

rLZ-8 (700μg/ml$^{-1}$) was prepared with normal saline into the preparation of 700μg·ml$^{-1}$; aluminium hydroxide (adjuvant) was prepared with PBS buffer into the preparation of 15mg •ml$^{-1}$; Bovine Serum Albumin (BSA) and ovalbumin (OVA).

**2. Experimental Method**

40 male mice (15 weeks old, weight of 18-22g), which were placed in circumstance of no heat source, were divided into 4 groups: group I of rLZ-8, group II of rLZ-8, positive control group, normal control group. Each mouse of group I of rLZ-8 was intraperitoneally injected with 0.1ml/10g body weight rLZ-8 twice a week, and injected 6 times totally; after injected 2 times, said mice were intraperitoneally injected with the mixture of BSA (1 mg) and 0.2 ml suspension of aluminium hydroxide (15mg •ml$^{-1}$) so as to sensitization; and they were injected 1mg BSA plus 0.2 ml PBS via tail vein on the seventeenth day of sensitization, and observed the allergic reaction. Each mouse in normal control group was administrated with isovolumetric normal saline replacing rLZ-8 . Each mouse of group II of rLZ-8 was injected with 0.1ml/10g body weight mixture of BSA-rLZ-8 (rLZ-8 70 μg • ml$^{-1}$ and BSA 5mg • ml$^{-1}$, prepared with PBS) via vein on the seventeenth day of sensitization. Each mouse in positive control group was injected with 1 mg OA replacing BSA via vein on the seventeenth day of sensitization. Criterion for observation: systemic allergic reaction was observed 30 min after injection via vein. Criterion judging for positive reaction: ashyperspasmia, reduction in activity or death. Criterion judging for negative reaction: normal activity.

As shown in table 14, rLZ-8 inhibited systemic allergic reaction in mice caused by BSA.When rLZ-8 and BSA in dose causing shock was injected synchronously, rLZ-8 did not prevent systemic allergic reaction.

**Table 14. The results of rLZ-8 inhibiting systemic allergic reaction in mice**

| Groups | Sensitization | Shock | rLZ-8 treatment | results | |
|---|---|---|---|---|---|
| | | | | Sensitization number/total | Death number/total |
| Normal contrast | BSA (i.p.) | BSA (i.v.) | - | 10/10 | 1/10 |
| Positive contrast | BSA (i.p.) | OA (i.v.) | - | 0/10 | 0/10 |
| rLZ-8dose1 | BSA (i.p.) | BSA (i.v.) | + | 5/10 | 0/10 |
| rLZ-8dose 2 | BSA (i.p.) | BSA- rLZ-8 (i.v.) | + | 9/10 | 0/10 |

**Example 13 rLZ-8 hemolysis test**

**1. Experimental Reagent**

rLZ-8 was prepares with 5% of glucose solution into the preparation of 1mg·ml$^{-1}$; blood cell suspension preparation: human blood 4ml, 1000r/min centrifugal 10min, clears supernatant. By adding 5% glucose solution about 10 times of the volume to erythrocyte sedimentation, shaking, centrifugal 1000r/min 20min, clear the supernatant, repeat the washing 2-3 times until the supernatant is not significantly red. Prepare the obtained erythrocyte with 5% glucose solution into 2% suspension for experiment.

## 2. Experimental Method

Get 28 clean test tubes and number them, No.1-5 for rLZ-8 drug group, No.6 for negative control group (5% glucose solution), No. 7 for positive control tube (distilled water), and a total of 4 parallel comparison tubes. By supplying 2% erythrocyte suspension, 5% glucose or distilled water in turn, shaking, put them into incubators $37°C \pm 0.5°C$ immediately for incubation. Observe them every 15min, 1h after observe them once for 3 hours. Finishing the observation, put all the solution from the tubes into centrifugal drying tubes, 1500r, 25min. Clear the supernatant, read the blank distilled water tube for OD value to calculate the rate of hemolysis.

## 3. Experimental Result

We can see in Table 15 that rLZ-8 hmolytic rate of drug group from 1-5 group <5%, there is no sign of hemolytic reaction.

**Table 15 Experiment results of rLZ-8 hemolysis on human erythrocyte solubilization ($\overline{X}\pm$s, n=10)**

| Serial number of test tubes | 1 | 2 | 3 | 4 | 5 |
|---|---|---|---|---|---|
| Hemolysis rate (%) | $0.70\pm0.03$ | $0.74\pm0.04$ | $0.63 \pm 0.04$ | $0.65\pm0.04$ | $0.59\pm0.07$ |

## Example 14: Influence of rLZ-8 on rat myelogram

### 1. Experimental Reagent

Waster rats 9, about 100g. Prepare rLZ-8 with sterile saline solution for three dose groups of 60mg·kg$^{-1}$, 30 mg·kg$^{-1}$, 15 mg·kg$^{-1}$.

### 2. Experimental Method

Normal control group 3 (rats), protein low-dose group 2, protein medium-dose group 2, and protein high-dose group 2. rLZ-8 drug group rats, are given different doses of rLZ-8 tail vein injection respectively, 1 time/day; the normal control group are given equivalent saline. On the 7th day of injection, check the right side of the thigh bone marrow for smears.

### 3. Experimental Result

Compare the rat bone marrow smear with the normal control group, no abnormal appears.

## Example 15: rLZ-8 on human red blood cell cohesion effects

### 1. Experimental Reagent

A, B, O and AB blood type, from the healthy volunteers 2ml respectively, SRBC (sheep red cell) 2ml. Centrifuge the above RBC 1200g·min$^{-1}$ for 10min, clears the supernatant and wash with 5ml PBS, repeat the above operation 3-5 times, and then uses 0.01mol/L PBS suspension preparation of 1.5%. Prepare rLZ-8 with normal saline to final concentrations of 50$\mu$g·ml$^{-1}$, 25$\mu$g·ml$^{-1}$, 12.5$\mu$g·ml$^{-1}$, 6.25$\mu$g·ml$^{-1}$, 3.13$\mu$g·ml$^{-1}$, 1.56$\mu$g·ml$^{-1}$, 0.78$\mu$g·ml$^{-1}$, 0.39$\mu$g·ml$^{-1}$, 0.20$\mu$g·ml$^{-1}$, 0.10$\mu$g·ml$^{-1}$, 0.05$\mu$g·ml$^{-1}$, 0.03$\mu$g·ml$^{-1}$. Plant agglutinin (PHA) preparation Ibid.

### 2. Experimental Method

Divide experiment subjects into rLZ-8 concentration groups, positive drug control, and normal control group. On 96-orifice Hemagglutination board, we add A-type 1.5%, erythrocyte 25$\mu$l/hole, and then add 0.2% gelatin, 75$\mu$l/hole. Drug groups are added different concentrations of rLZ-8, till the final concentration as above-noted; positive drug control group PHA 25$\mu$l/hole; normal control group PBS25$\mu$l/hole. 6 parallel control groups in each group. Shaking for 30s under normal room temperature, 37°C incubation, observe the subjects 1h later. For B-type, AB-type, O-type, the same experiment methods are as SRBC (sheep red cell) above.

## 3. Experimental Result

Table 16 below indicates that positive drug PHA is agglutinative on all four types of human red blood cells and sheep red blood cells; rLZ-8 is not agglutinative on four types of human red blood cells, while at $12.5\mu g\cdot ml^{-1}$-$50\mu g\cdot ml^{-1}$ concentration SRBC shows active agglutination.

**Table 16 rLZ-8 Experiment result of 4 types of human red blood cell agglutination**

| grouping | Normal group | rLZ-8 medicine group (µg·ml$^{-1}$) | | | | | | | | | | | |
|---|---|---|---|---|---|---|---|---|---|---|---|---|---|
| | | 50 | 25 | 12.5 | 6.25 | 3.13 | 1.56 | 0.78 | 0.39 | 0.20 | 0.10 | 0.05 | 0.03 |
| A | - | - | - | - | - | - | - | - | - | - | - | - | - |
| B | - | - | - | - | - | - | - | - | - | - | - | - | - |
| O | - | - | - | - | - | - | - | - | - | - | - | - | - |
| AB | - | - | - | - | - | - | - | - | - | - | - | - | - |
| SRBC (sheep red cell) | - | +++ | ++ | + | - | - | - | - | - | - | - | - | - |

**Example 16: The Recombinant Ganoderma Lucidum Immune Regulatory Protein anti-tumor preparation**

Through above pharmacological experiments, it proves that the effect of Recombinant Ganoderma Lucidum Immune Regulatory Protein rLZ-8 anti-tumor and raising level of interleukin is very significant, and non-toxic without side-effect. Therefore, it can be said that the Recombinant Ganoderma Lucidum Immune Regulatory Protein rLZ-8 as an anti-tumor preparation is suitable for drug use and is safe.

This invention of Recombinant Ganoderma Lucidum Immune Regulatory Protein rLZ-8 as an application of anti-cancer drug can be given orally and non-intestinal drug delivery. Dosage can be decided by symptoms, age, weight and other factors. For adults, the oral dosage for per person is 10-1000mg, several times a day; non-intestinal delivery of 10-100mg, several times a day.

In this invention, we have kits of oral tablets, pills and capsules (including the hard and soft capsules), these preparation formulations include the Recombinant Ganoderma Lucidum Immune Regulatory Protein rLZ-8 and at least one inert diluent (such as lactose, mannose alcohol, glucose, starch, poly vinyl pyrrolidone), it can also be joined by acceptable additives such as lubricants, disintegrants, stabilizers other than the inert diluent pharmacology. If it is necessary, tablets or pills can be coated by gastric soluble or entric coating material on one or more than one layer of coatings. Non-intestinal injection includes Recombinant Ganoderma Lucidum Immune Regulatory Protein rLZ-8 and at least one inert water diluent (such as distilled injection water and normal saline), Recombinant Ganoderma Lucidum Immune Regulatory Protein rLZ-8 can also be made freeze-dried powder; we may dissolve the powder in inert diluent water for injection before use.

**(1) Preparation case 1**

Get Recombinant Ganoderma Lucidum Immune Regulatory Protein rLZ-8 1000mg; dissolve in 100ml of sterile saline, mixing evenly, then sub-pack them into rLZ-8 10mg/ml/piece concentration solution injection bottle; sealed, sterilized and made to be products. Other items shall conform to the pharmacopoeia of the People's Republic of China 2005 edition under the requirements of injection.

**(2) Preparation case 2**

Get Recombinant Ganoderma Lucidum Immune Regulatory Protein rLZ-8 100g; medicinal starch 0.5kg, in accordance with the publicly-known technology and equipment manufacture them into capsule forms, rlz8 10mg/tablets and other items that shall conform to the pharmacopoeia of the People's Republic of China 2005 edition under the requirements of capsules.

**(3) Preparation case 3**

Get Recombinant Ganoderma Lucidum Immune Regulatory Protein rLZ-8 100g; microcrystalline cellulose 560g; anhydrous lactose 380g; magnesium stearate 200g, in accordance with the publicly-known technology and equipment manufacture them into pills/tablets, rLZ-8 10mg/tablet, other itmes shall conform to the pharmacopoeia of the the People's Republic of China 2005 edition under the requirements of tablets.

**(4) Preparation Case 4**

Get Recombinant Ganoderma Lucidum Immune Regulatory Protein rLZ-8 moderate, items shall conform to the pharmacopoeia of the People's Republic of China 2005 edition under the requirements of oral liquid, manufacture oral liquid in accordance with the publicly-known technology and equipment.

**Industrial Applicability**

Results of the present invention indicate that recombinant Ganoderma Lucidum Immunoregulatory Protein has notable effects on treating and preventing hypoleukocytosis caused by various reasons, and is superior to the existing clinical drugs in clinical dose, treating effects and clinical discomfort. Animal tests show that the adverse effects of recombinant Ganoderma Lucidum Immunoregulatory Protein is far lower than recombinant colony-stimulating factors.

The inventor has succeeded in: expressing Ganoderma Lucidum Immunoregulatory Protein using eukaryote expression system of Pichia pastoris; realizing, among the similar researches in the world, for the first time 100 L fermenting production for rLZ-8, and establishing the process for production and purification; and completing for the first time the determination of spatial structure of rLZ-8 and thus providing an excellent foundation for further research on rLZ-8's anti-tumor mechanism.

The invention innovatively finds that: rLZ-8 is effective in increasing the number of leukocytes of animal models with low lymphocyte level and has obviously better pharmacodynamic effect than colony stimulating factors; rLZ-8 can induce apoptosis of leukemia cells Nb4, K562 and HL-60 (we draw, for the first time, the conclusion that this is another possible ways for fungal immunomodulatory protein to kill tumor cells). The treatment experiment on tumor-bearing mice further indicate that rLZ-8 is effective in inhibiting growth of transplanted tumor *in vivo;* the safety experiments showed that rLZ-8 does not have obvious virulence on rat' blood cells and does not impose pathologic destructive effect on vascular wall of aorta and cardinal vein; and the results of bone marrow smear indicated that rLZ-8 does not have pathogenic effect on hematopoietic function. The present invention disclose for the first time the crystal spatial structure of LZ-8, discloses that LZ-8 can directly kill the tumor cells without damaging the normal cells, and discloses the new use of LZ-8 in preventing and treating hypoleukocytosis caused by various reasons.

**Claims**

1. Use of a recombinant Ganoderma Lucidum Immunoregulatory Protein (rLZ-8) encoded by a nucleotide sequence **SEQ ID NO 1** in preparation of medicaments for treating a cancer selected from the group consisting of leukemia, ascites tumor and liver cancer.

2. The use according to claim 1, wherein the amino acid sequence of the said recombinant Ganoderma Lucidum Immunoregulatory Protein (rLZ-8) is MSDTALIFRLAWDVKKLSFDYTPNWGRGNPNNFIDTVTFPKVLTDK VF WDPDTNNDFIIAQWN, comprising an important N-terminal domain needed for the formation of dimerization and a C-terminal FNIII domain;
   the said N-terminal domain being composed of an α-helix having an amino acid sequence of 2-SDTALIFRLAWDVK-15 and consisting of 14 amino acids, and a β-strand having an amino acid sequence of 16-KLSFD-20 and consisting of 5 amino acids, wherein Serine residues on said α-helix are blocked by acetylation;
   the N-terminal α-helix and β-strand of one LZ-8 monomer forming, together with the same domains of another LZ-8 monomer, an important dumb-bell-shaped dimmer-binding domain via domain swapping;
   the C-terminal FNIII domain belonging to an immunoglobulin-like sandwich structure and comprising β-sheet I and β-sheet II formed by β-strands A-B-E and β-strands G-F-C-D, respectively; wherein the sequence of β-strand was A. 21-TPNWGRG-27; B. 34-IDTVTFP-39; C. 48-YTYRVAV-54;D. 57-RNLGVKP-63; E. 72-SQKVN-76; F. 91-TIQVFVVDPD-100; G. 102-NNDFIIAQW-110.

3. The use according to any one of claims 1-2, wherein the said medicament comprises the recombinant Ganoderma Lucidum Immunoregulatory Protein (rLZ-8) and a pharmaceutically acceptable adjuvant.

4. The use according to claim 3, wherein the said medicament may be orally administrated or parenterally administrated.

5. The use according to claim 4, wherein the orally administered medicament includes tablet, pill and capsule.

6. The use according to claim 4, wherein the parenterally administered medicament includes drug for external use and injection.

7. The use according to claim 6, wherein injection includes freeze-dried powder injection and water injection.

**Patentansprüche**

1. Verwendung eines rekombinanten Ganoderma Lucidium Immunregulationsproteins (rLZ-8), codiert durch eine Nukleotidsequenz SEQ ID NO 1, bei der Zubereitung von Medikamenten für die Behandlung einer Krebserkrankung, die ausgewählt ist aus der Gruppe bestehend aus Leukämie, Aszites-Tumor und Leberkrebs.

2. Verwendung nach Anspruch 1, bei der die Aminosäuresequenz des rekombinanten Ganoderma Lucidium Immunregulationsproteins (rLZ-8) MSDTALIFRLAWDVKKLSFDYTPNWGRGNPNNFIDTVTFPKVLTDK VF WDPDTNNDFIIAQWN ist, mit einer wesentlichen N-End-Domäne, die für die Bildung der Dimerisation benötigt wird, und einer C-End-FNIII-Domäne;
   wobei die N-End-Domäne aufgebaut ist aus einer α-Helix mit einer Aminosäuresequenz 2-SDTALIFRLAWDVK-15 und bestehend aus 14 Aminosäuren, und einem β-Strang mit einer Aminosäuresequenz 16-KLSFD-20 und bestehend aus 5 Aminosäuren, wobei Serin-Reste auf der α-Helix durch Acetylierung blockiert sind;

die N-End-α-Helix und der β-Strang eines LZ-8 Monomers zusammen mit denselben Domänen anderer LZ-8-Monomere eine wesentliche hantelförmige dimerbindende Domäne durch Domänenaustausch bilden; die C-End-FNIII-Domäne zu einer immunglobulinartigen Sandwichstruktur gehört und einen β-Strang I und einen β-Strang II aufweist, die gebildet sind durch β-Stränge A-B-E bzw. β-Stränge G-F-C-D; wobei die Sequenz des β-Stranges A. 21-TPNWGRG-27; B. 34-IDTVTFP-39; C. 48-YTYRVAV-54; D. 57-RNLGVKP-63; E. 72-SQKVN-76; F. 91-TIQVFVVDPD-100; G. 102-NNDFIIAQW-110 war.

3. Verwendung nach Anspruch 1 oder 2, bei der das Medikament das rekombinante Ganoderma Lucidium Immunregulationsprotein (rLZ-8), und ein pharmazeutisch akzeptables Adjuvans aufweist.

4. Verwendung nach Anspruch 3, bei der das Medikament oral oder parenteral verabreicht werden kann.

5. Verwendung nach Anspruch 4, bei der das oral verabreichte Medikament Tabletten, Pillen und Kapseln umfasst.

6. Verwendung nach Anspruch 4, bei der das parenteral verabreichte Medikament eine Arznei für externe Anwendung und Injektion umfasst.

7. Verwendung nach Anspruch 6, bei der die Injektion eine Injektion von gefriergetrocknetem Pulver und eine Wasser-Injektion umfasst.

**Revendications**

1. Utilisation d'une protéine immunorégulatrice recombinante de Ganoderma Lucidum (rLZ-8) codée par une séquence de nucléotides SEQ ID NO: 1 dans la préparation de médicaments pour traiter un cancer sélectionné dans le groupe consistant en la leucémie, une tumeur ascitique et le cancer du foie.

2. Utilisation selon la revendication 1, dans laquelle la séquence d'acides aminés de ladite protéine immunorégulatrice recombinante de Ganoderma Lucidum (rLZ-8) est MSDTALIFRLAWDVKKLSFDYTPNWGRGNPNNFIDTVTFPKVLTDKVFWDPDTNNDF IIAQWN, comprenant un important domaine N-terminal nécessaire pour la formation d'une dimérisation et un domaine FNIII C-terminal ; ledit domaine N-terminal étant composé d'une hélice α possédant une séquence d'acides aminés de 2-SDTALIFRLAWDVK-15 et consistant en 14 acides aminés, et d'un brin β possédant une séquence d'acides aminés de 16-KLSFD-20 et consistant en 5 acides aminés ; où les résidus sérine sur ladite hélice α sont bloqués par une acétylation ; l'hélice α et le brin β N-terminaux d'un monomère LZ-8 formant, avec les mêmes domaines d'un autre monomère LZ-8, un important domaine en forme d'haltère se liant à un dimère via une permutation de domaines ; le domaine FNIII C-terminal appartenant à une structure en sandwich de type immunoglobuline et comprenant un feuillet β I et un feuillet β II formés par des brins β A-B-E et des brins β G-F-C-D, respectivement ; où la séquence du brin β était A. 21-TPNWGRG-27 ; B. 34-IDTVTFP-39 ; C. 48-YTYRVAV-54 ; D. 57-RNLGVKP-63 ; E. 72-SQKVN-76 ; F. 91-TIQVFVVDPD-100 ; G. 102-NNDFIIAQW-110.

3. Utilisation selon l'une quelconque des revendications 1 à 2, dans laquelle ledit médicament comprend la protéine immunorégulatrice recombinante de Ganoderma Lucidum (rLZ-8) et un adjuvant pharmaceutiquement acceptable.

4. Utilisation selon la revendication 3, dans laquelle ledit médicament peut être administré par voie orale ou administré par voie parentérale.

5. Utilisation selon la revendication 4, dans laquelle le médicament administré par voie orale comprend un comprimé, une pilule et une capsule.

6. Utilisation selon la revendication 4, dans laquelle le médicament administré par voie parentérale comprend un médicament pour un usage externe et une injection.

7. Utilisation selon la revendication 6, dans laquelle l'injection comprend une injection de poudre lyophilisée et une injection d'eau.

**Figure 1.   rLZ-8 crystal structure captions**

Note 1: The blue one and the yellow one indicates a rLZ-8 monomer, respectively;

Note 2: A, B, C, D, E, F and G indicates a β-strand, respectively;

Note 3: N-term indicates N terminal of rLZ-8, and the C-term indicates C terminal of rLZ-8

**Figure2. rLZ-8 on NB4 tumor cells in vitro results**

Note： A： Normal control group； B： Positive drug control group； C-J： rLZ-8 groups follow the order 0.78µg·ml⁻¹, 1.56µg·ml⁻¹, 3.125µg·ml⁻¹, 6.25µg·ml⁻¹, 12.5µg·ml⁻¹, 25µg·ml⁻¹, 50µg·ml⁻¹, 100µg·ml⁻¹

**Figure 3. rLZ-8 on K562 tumor cells in vitro results**

Note：A：Normal control group；B：Positive drug control group；C-H：rLZ-8 groups follow the order 3.125μg·ml⁻¹, 6.25μg·ml⁻¹, 12.5μg·ml⁻¹, 25μg·ml⁻¹, 50μg·ml⁻¹, 100μg·ml⁻¹

**Figure 4 rLZ-8-induced apoptosis of K562 and NB4 cells, PI single staining test results**

Note 1： C1： K562 Normal control group； I1： rLZ-8 group （K562， 0.1 μ g/ml）； I2： rLZ-8 group （K562， 0.5 μ g/ml）； I3： rLZ-8 group （K562， 2.5 μ g/ml）

Note 2： C2： NB4 Normal control group； H1： rLZ-8 group （NB4， 0.1 μ g/ml）； H2： rLZ-8 group （NB4， 0.5 μ g/ml）； H3： rLZ-8 group （NB4， 2.5 μ g/ml）

**Figure 5 rLZ-8-induced apoptosis of HL-60 and NB4 cells, Annexin V/PI double staining test results**

Note 1: C1: NB4 Normal control group; P1: Positive drug control group; I1: rLZ-8 group (NB4, 0.1 µ g/ml); I2: rLZ-8 group (NB4, 0.5 µ g/ml); I3: rLZ-8 group (NB4, 2.5 µ g/ml)

Note 2: C2: NB4 Normal control group; P2: Positive drug control group; H1: rLZ-8 group (HL-60, 0.1 µ g/ml); H2: rLZ-8 group (HL-60, 0.5 µ g/ml); H3: rLZ-8 group (HL-60, 2.5 µ g/ml)

**Figure 6 inoculated S180 Ehrlich ascites tumor cells in mice body weight change**

**Figure 7 inoculated H22 tumor cells implanted in mice body weight change**

Figure 8. FITC-rLZ-8 (100ng.ml$^{-1}$) rat myocardial tissue markers (dark, DIC field)

Figure 9 FITC-rLZ-8 (100ng.ml$^{-1}$) rabbit chondrocyte markers (dark, DIC field)

Figure 10 FITC-rLZ-8 (100ng.ml$^{-1}$) HL-60 cell markers (dark, DIC field)

**Figure 11 Rat myelogram influence by rLZ-8**

Note：A：Normal control group ；B：rLZ-8（15 mg·kg$^{-1}$）；C：rLZ-8（30 mg·kg$^{-1}$）；
D：rLZ-8（60 mg·kg$^{-1}$）

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **KOHSUKE KINO et al.** *J. Bio. Chem.,* 1989, vol. 1, 472-478